# EUROPÄISCHE PATENTSCHRIFT

(11) **EP 3 854 473 B1**
(45) Veröffentlichungstag und Bekanntmachung des Hinweises auf die Patenterteilung: **01.03.2023**
(21) Anmeldenummer: 20153505.1
(22) Anmeldetag: 24.01.2020
(51) Int. Cl.: B01F 23/70, B01F 33/841

(54) **ABTÖNANLAGE FÜR EIN KIT-OF-PARTS-ABTÖNSYSTEM, VERFAHREN ZUR HERSTELLUNG EINES ABGETÖNTEN ANSTRICH- ODER PUTZSYSTEMS SOWIE VERWENDUNG DER ABTÖNANLAGE**
TINTING SYSTEM FOR A KIT-OF-PARTS TINTING SYSTEM, METHOD FOR PRODUCING A TINTED PAINT OR PLASTER SYSTEM AND USE OF THE TINTING SYSTEM
INSTALLATION À TEINTER POUR UN SYSTÈME À TEINTER EN KIT, PROCÉDÉ DE FABRICATION D'UN SYSTÈME DE PEINTURE OU DE NETTOYAGE TEINTÉ AINSI QU'UTILISATION DE L'INSTALLATION À TEINTER

(43) Veröffentlichungstag der Anmeldung: 28.07.2021
(73) Patentinhaber: DAW SE, 64372 Ober-Ramstadt (DE)
(72) Erfinder: Weber, Enno, 64372 Ober-Ramstadt (DE); Karner, Karl, 64665 Alsbach-Hähnlein (DE)
(74) Vertreter: Metten, Karl-Heinz

(56) Entgegenhaltungen:
- EP-A1- 2 661 320
- US-A1- 2018 368 558

## Beschreibung

Die vorliegende Erfindung betrifft eine Abtönanlage für ein Kit-of-Parts-Abtönsystem, enthaltend eine Abtönbasiszusammensetzung und mindestens eine Farbton- oder Extenderzusammensetzung. Des Weiteren betrifft die Erfindung ein Verfahren zur Herstellung eines abgetönten Anstrich- oder Putzsystems mit der Abtönanlage sowie die Verwendung der Abtönanlage.

Die Nachfrage nach unterschiedlichsten Anstrichfarben oder gefärbten Putzen für die Innen- oder Außenanwendung ist in Zeiten, wo individuelles Gestalten und einzigartige Wohnkonzepte hohen Anklang finden, immens. Die Farbpaletten bieten heute schon Abstufungen in kleinsten Nuancen, sodass es wenig zeitgemäß und wirtschaftlich ist, Anstrichfarben und Putze jeglicher Schattierung in großen Mengen herzustellen und auf Vorrat zu lagern. Vielmehr haben sich in den letzten Jahren Abtönsysteme auf dem Markt durchgesetzt, welche aus einer Abtönbasiszusammensetzung, meist in weißer oder transparenter Form, und einer oder mehrerer Farbton- oder Extenderzusammensetzungen bestehen, die vermischt werden. Die Mischung der einzelnen Komponenten sowie die genaue Farbtoneinstellung werden heutzutage über softwaregesteuerte Abtönanlagen erzielt. Eine Vielzahl von Farbton- oder Extenderzusammensetzungen sind auf Wasserbasis. Wird in diesen Zusammensetzungen auf Konservierungsmittel verzichtet, die neben Wasser noch eine Vielzahl organischer Verbindungen enthalten, besteht eine besonders hohe Gefahr des Bakterienbefalls und letztlich auch der Schimmelbildung, da beim Abfüllen in der Abtönanlage regelmäßig Kontakt mit der Umgebungsluft besteht. Die so verunreinigten Zusammensetzungen müssen entsorgt und die Abtönanlage zeitaufwendig und kostenintensiv gereinigt werden. Während dieser Zeit steht die Abtönanlage still.

Das Problem des Bakterienbefalls sowie der Schimmelbildung wird gemäß dem Stand der Technik über den Zusatz von Bioziden in Abtönpasten minimiert bzw. gelöst. Allerdings ist es unter Gesundheitsaspekten wie auch aus umwelttechnischen Gründen wünschenswert, auf Biozide zu verzichten. Daher ist es also nötig, neue Wege und Methoden zu finden, welche Abtönanlagen keimfrei halten und somit den Bakterienbefall in diesen Anlagen minimieren helfen sowie die Schimmelbildung in den Zusammensetzungen zu verhindern.

Die EP 3 395 454 A1 offenbart ein Ausgabesystem für insbesondere konservierungsmittelfreie Farbtonpasten, wobei das Ausgabesystem Reinigungsmittel und -techniken umfasst, welche die möglichen Mikroorganismen neutralisieren sollen. Eine Möglichkeit sei die innere Oberfläche des Ausgabesystems in Form der Kanister, Ausgabedüsen und Rührer als ultrahydrophob beschichtete Oberfläche auszubilden, um einen Lotus-Effekt zu erzielen. Die Beschichtungen können dabei biozide Mittel, metallisches Pulver, ANA (*advanced nano solutions*) Additive oder Silikonbeschichtungen umfassen. Weiterhin erwähnt die EP 3 395 454 A1 das Spülen des Ausgabesystems mit einem inerten Gas, den Zusatz von Reinigungsmitteln in Form von Konservierungsmitteln in den Farbpasten oder den Basislacken und den Einsatz von anti-mikrobiologischen Filtern in den Füllöffnungen der Farbkanister.

Die gezeigten Möglichkeiten weisen aber die bereits erwähnten Nachteile der Umwelt- und Gesundheitsbelastung beim Einsatz von Bioziden in den Zusammensetzungen auf. Weiterhin stellen die eingesetzten Filtersysteme eine weitere Systemkomponente dar, die anfällig für einen Bakterienbefall und/oder Schimmelbildung sein könnte. Die regelmäßige Spülung des Systems mit Inertgas ist neben dem Kostenfaktor zusätzlich ein Systemparameter, der überwacht und gewartet werden muss.

EP 2661320 B1 offenbart eine Abtönanlage gemäß dem Oberbegriff des Anspruch 1. US20180368558 offenbart einen Kosmetikspender mit UV Strahlungsquelle in den individuellen Zutatenbehältern.

Der vorliegenden Erfindung lag daher die Aufgabe zugrunde, Abtönanlagen bereitzustellen, die nicht mit den Nachteilen des Stands der Technik behaftet sind und auch für Kit-of-Parts Abtönsysteme auf Wasserbasis, insbesondere für biozidfreie oder nahezu biozidfreie Kit-of-Parts Abtönsysteme auf Wasserbasis, geeignet sind, ohne den Befall mit Bakterien und/oder Schimmelbildung gerade auch über einen längeren Zeitraum befürchten zu müssen.

Demgemäß wurde eine Point-of-Sale- oder eine Point-of-Use-Abtönanlage gemäß dem Wortlaut des Anspruch 1 für ein Kit-of-Parts-Abtönsystem enthaltend eine Abtönbasiszusammensetzung und mindestens eine Farbton- oder Extenderzusammensetzung gefunden, umfassend einen Vorratsbehälter für eine Farbton- oder Extenderzusammensetzung oder eine Vielzahl an Vorratsbehältern für eine Vielzahl an Farbton- oder Extenderzusammensetzungen und
gegebenenfalls einen, zwei oder mehr Vorratsbehälter für eine Abtönbasiszusammensetzung oder für Komponenten einer Abtönbasiszusammensetzung,
wobei der oder die Vorratsbehälter mindestens einen Behältereinlass, eine Behälterwandung, umfassend eine Behälterinnenwand, einen Behälterboden und einen Behälterauslass aufweisen, gekennzeichnet durch
mindestens eine, insbesondere 2, 3, 4 oder mehr UV-Strahlungsquellen, die dem einen Vorratsbehälter zuordenbar ist bzw. sind oder
mindestens eine, insbesondere 2, 3, 4 oder mehr UV-Strahlungsquellen, die mindestens zwei Vorratsbehältern, insbesondere sämtlichen, der Vielzahl an Vorratsbehältern zuordenbar ist bzw. sind oder
mindestens eine, insbesondere 2, 3, 4 oder mehr UV-Strahlungsquellen, die jeweils einem der Vielzahl an Vorratsbehältern zuordenbar ist bzw. sind,
wobei die mindestens eine, insbesondere 2, 3, 4 oder mehr UV-Strahlungsquellen ausgelegt und eingerichtet ist bzw. sind, um die Behälterinnenwand der Behälterwandung mindestens eines Vorratsbehälters, insbesondere eines Vorratsbehälters, mindestens abschnittsweise sowie gegebenenfalls auch den Behältereinlass, den Behälterboden, in dem Behälter vorliegendes Zubehör, insbesondere ein Rührwerkzeug, eine in dem Vorratsbehälter vorliegende Farbton- oder Extenderzusammensetzung und/oder den Behälterauslass mindestens abschnittsweise mit UV-Strahlung zu bestrahlen.

Ferner kann die Abtönanlage mindestens einen Vorratsbehälter für die Abtönbasiszusammensetzung mit einem Behältereinlass, einer Behälterwandung, einem Behälterboden und einem Behälterauslass enthalten. Ist der Vorratsbehälter für die Abtönbasiszusammensetzung in der Abtönanlage ebenfalls vorhanden, lässt sich in der Anlage die fertige Anstrichfarbe oder der Putz benutzungsfertig anmischen. Diese Ausführungsform einer erfindungsgemäßen Abtönanlage eignet sich insbesondere für die industrielle Fertigung von abgetönten Beschichtungsmassen.

In einer weiteren, besonders geeigneten Ausführungsform einer erfindungsgemäßen Abtönanlage ist vorgesehen, dass die jeweils mit den Behälterauslässen der Vielzahl an Vorratsbehältern für die Farbton- oder Extenderzusammensetzungen oder die mit den jeweils einem Vorratsbehälter zugeordneten Dosierpumpen in Verbindung stehenden weiteren Fließkanäle eingerichtet und ausgelegt sind, um die Farbton- oder Extenderzusammensetzungen einem Mischbehälter zum Vermengen mit einer darin vorlegbaren oder insbesondere vorliegenden Abtönbasiszusammensetzung zuzuführen. Bei der vorangehend geschilderten Ausführungsvariante verfügt die erfindungsgemäße Abtönanlage vorzugsweise nicht über einen Vorratsbehälter für die Abtönbasiszusammensetzung, der zusätzlich zu den Behältereinlass, der Behälterwandung und dem Behälterboden auch einem Behälterauslass ausgestattet ist. Diese Variante einer erfindungsgemäßen Abtönanlage verfügt zweckmäßigerweise nicht mehr über einen Vorratsbehälter für die Abtönbasiszusammensetzung, vielmehr kann die Abtönbasiszusammensetzung in dem Mischbehälter vorgelegt werden. Für viele Anwendungen hat es sich als besonders zweckmäßig erwiesen, dass der Mischbehälter sogleich das vor Ort zu nutzende Gebinde bzw. das Verkaufsgebinde, beispielsweise einen Farbeimer, darstellt. Die vorangehende Ausführungsvariante eignet sich demgemäß insbesondere für den Einsatz am Point-of-sale oder am Point-of-use. D.h. die mit dieser Ausführungsform einer erfindungsgemäßen Abtönanlage durch Mischen von Farbton- bzw. Extenderzusammensetzung und Abtönbasiszusammensetzung erhaltenen abgetönten Beschichtungsmassen können direkt verwendet werden.

In bevorzugten Ausgestaltungsformen erfindungsgemäßer Abtönanlagen liegt die Vielzahl an Vorratsbehältern für die mindestens eine Farbton- oder Extenderzusammensetzung sowie gegebenenfalls ein, zwei oder mehr Vorratsbehälter für eine Abtönbasiszusammensetzung oder für Komponenten einer Abtönbasiszusammensetzung auf einem, insbesondere rotierbaren, Karussell angeordnet vor. Die Zugabe von mehreren unterschiedlichen Farbton- oder Extenderzusammensetzungen zu der Abtönbasiszusammensetzung kann auf diese Weise schnell und platzsparend erfolgen. Dabei umfasst die Anzahl an Vorratsbehältern für die Farbton- oder Extenderzusammensetzungen in einer zweckmäßigen Ausgestaltung der erfindungsgemäßen Abtönanlage einen Bereich von 16 bis 32 Vorratsbehälter, besonders bevorzugt sind erfindungsgemäßen Abtönanlagen mit 20 bis 32 Vorratsbehältern. Mit derartigen erfindungsgemäßen Abtönanlagen lässt sich eine Vielzahl unterschiedlicher Farbtöne kreieren.

Für das Vermengen von Abtönbasiszusammensetzung und Farbton- bzw. Extenderzusammensetzung in dem Mischbehälter kann auf geeignete Mischaggregate zurückgegriffen werden. Besonders geeignet sind zum Beispiel Rüttelmaschinen wie Rüttelplatten, gyroskopische Mischer oder mechanische Mischer wie Rührer.

Die erfindungsgemäße Abtönanlage umfasst ebenfalls einen Behälterverschlussdeckel für einen Vorratsbehälter für die Farbton- oder Extenderzusammensetzung, insbesondere eine Vielzahl an Behälterverschlussdeckeln für die Vielzahl an Vorratsbehältern, welche jeweils eingerichtet und ausgelegt sind, um den Vorratsbehälter reversibel zu verschließen, umfassend einen Deckelrand mit einer Innen- und einer Außenseite und vorzugsweise eine sich an den Deckelrand anschließende Deckelseitenwand mit einer Innen- und einer Außenseite, und eine mit dem Deckelrand oder der Deckelseitenwand verbundene Abdeckfläche mit einer Innen- und einer Außenseite, wobei die Innenseite des Deckelrandes und/oder die Innenseite der Deckelseitenwand und/oder die Innenseite der Abdeckfläche, insbesondere die Innenseite der Abdeckfläche, die mindestens eine UV-Strahlungsquelle, insbesondere 2, 3 oder 4 UV-Strahlungsquellen, umfasst, die ausgelegt und eingerichtet ist bzw. sind, um die Behälterinnenwand der Behälterwandung des Vorratsbehälters mindestens abschnittsweise sowie gegebenenfalls auch den Behältereinlass, die Innenseite der Deckelseitenwand, den Behälterboden, in dem Behälter vorliegendes Zubehör, insbesondere ein Rührwerkzeug, eine in dem Vorratsbehälter vorliegende Farbton- oder Extenderzusammensetzung und/oder den Behälterauslass mindestens abschnittsweise, insbesondere im Wesentlichen vollflächig, mit UV-Strahlung zu bestrahlen. Besonders bevorzugt sind die UV-Strahlungsquelle bzw. -quellen in dem Behälterverschlussdeckel eingerichtet und ausgelegt, um die Behälterinnenwand der Behälterwandung des Vorratsbehälters mindestens abschnittsweise, in dem Behälter vorliegendes Zubehör, insbesondere ein Rührwerkzeug, und eine in dem Vorratsbehälter vorliegende Farbton- oder Extenderzusammensetzung sowie gegebenenfalls auch den Behältereinlass, die Innenseite der Deckelseitenwand, den Behälterboden und den Behälterauslass mindestens abschnittsweise, insbesondere im Wesentlichen vollflächig, mit UV-Strahlung zu bestrahlen.

Im Falle des Vorratsbehälters für die Abtönbasiszusammensetzung in der Abtönanlage kann diese in einer zweckmäßigen Ausgestaltung ebenfalls mindestens eine UV-Strahlungsquelle umfassen, die dem mindestens einen Vorratsbehälter für die Abtönbasiszusammensetzung zuordenbar oder zugeordnet ist und die ausgelegt und eingerichtet ist, um die Behälterwandung sowie gegebenenfalls auch den Behältereinlass, den Behälterboden und/oder den Behälterauslass, mindestens abschnittsweise mit UV-Strahlung zu bestrahlen.

Um die Oberflächen möglichst effektiv keimfrei zu halten, hat sich als besonders geeignet erwiesen, wenn die mindestens eine UV-Strahlungsquelle im oberen Drittel der Behälterwandung des Vorratsbehältnisses, insbesondere im oberen Fünftel der Behälterwandung, vorliegt. Dies gilt für den Vorratsbehälter der Abtönbasiszusammensetzung.

In einer vorteilhaften Ausgestaltung der erfindungsgemäßen Abtönanlage umfasst oder enthält diese ferner
mindestens einen mit dem Behälterauslass des mindestens einen Vorratsbehälters der Farbton- oder Extenderzusammensetzungen in Verbindung stehenden weiteren Fließkanal. Besonders bevorzugt umfasst oder enthält die Abtönanlage mindestens einen jeweils mit den Behälterauslässen der Vielzahl an Vorratsbehältern für die Farbton- oder Extenderzusammensetzungen in Verbindung stehenden weiteren Fließkanal, jeweils mit Innenwandungen. Darüber hinaus kann auch ein mit dem Behälterauslass des Vorratsbehälters für die Abtönbasiszusammensetzung in Verbindung stehender Fließkanal mit einer Innenwandung vorgesehen sein.

Alternativ oder zusätzlich enthält oder umfasst die erfindungsgemäße Abtönmaschine mindestens einen Mischbehälter mit einem Behältereinlass, einer Behälterwandung, einem Behälterboden und einem Behälterauslass zum Vermengen der Abtönbasiszusammensetzung mit der mindestens einen Farbton- oder Extenderzusammensetzung. In dieser Ausführungsvariante stellt der Mischbehälter regelmäßig kein Verkaufsgebinde oder ein für die Benutzung vor Ort vorgesehenes Gebinde dar, sondern eignet sich vielmehr eher für die industrielle Fertigung und Abfüllung.

In einer zweckmäßigen Ausgestaltungsform werden die mindestens eine Farbton- oder Extenderzusammensetzung in dem mindestens einen Mischbehälter mit der Abtönbasiszusammensetzung vermischt.

Besonders vorteilhafte erfindungsgemäße Abtönanlagen verfügen über mindestens eine Dosierpumpe, die über einen Fließkanal mit dem Behälterauslass des mindestens einen Vorratsbehälters einer Farbton- oder Extenderzusammensetzung verbunden ist. Diese Dosierpumpe ist zweckmäßigerweise mit der Steuereinheit verbunden. Mithilfe der Dosierpumpe gelingt regelmäßig eine feinjustierte Abtönung der Abtönbasiszusammensetzung mit einer oder mit mehreren Farbton- oder Extenderzusammensetzungen,

Für die effektive Bekämpfung des Bakterienbefalls und/oder der Schimmelbildung haben sich auch solche Abtönanlagen als geeignet erwiesen, die mindestens eine UV-Strahlungsquelle aufweisen, die dem Fließkanal zuordenbar ist und die ausgelegt und eingerichtet ist, um die Innenwandung des Fließkanals mindestens abschnittsweise mit UV-Strahlung zu bestrahlen.

Alternativ oder zusätzlich ist es besonders vorteilhaft, wenn die erfindungsgemäße Abtönanlage mindestens eine weitere UV-Strahlungsquelle umfasst, die mindestens einem der weiteren, insbesondere jeweils einem der weiteren Fließkanäle, zuordenbar ist und die ausgelegt und eingerichtet ist, um die Innenwandung des mindestens einen weiteren Fließkanals mindestens abschnittsweise mit UV-Strahlung zu bestrahlen.

Ebenfalls kann in geeigneten Ausführungsformen auf Abtönanlagen zurückgegriffen werden, bei denen alternativ oder zusätzlich mindestens eine weitere UV-Strahlungsquelle, dem Mischbehälter zuordenbar ist und die ausgelegt und eingerichtet ist, um die Behälterwandung des Mischbehälters mindestens abschnittsweise mit UV-Strahlung zu bestrahlen.

In weiteren zweckmäßigen Ausgestaltungen der erfindungsgemäßen Abtönanlagen kann alternativ oder zusätzlich vorgesehen sein, dass die mindestens eine UV-Strahlungsquelle innerhalb des Vorratsbehälters für die Farbton- oder Extenderzusammensetzung und gegebenenfalls auch innerhalb des Vorratsbehälters für die Abtönbasiszusammensetzung ortsveränderbar angeordnet ist und beispielsweise einen konstanten Abstand zu der Oberfläche der in dem jeweiligen Vorratsbehälter vorliegenden Farbton- oder Extenderzusammensetzung bzw. Abtönbasiszusammensetzung beibehält. D.h. die mindestens eine UV-Strahlungsquelle kann auch eingerichtet und ausgelegt sein, um sich dem ändernden Pegelstand der in dem jeweiligen Vorratsbehälter vorliegenden Zusammensetzungsmasse anzupassen.

Die erfindungsgemäße Abtönanlage kann in einer weiteren, sehr zweckmäßigen Ausführungsform auch ausgestattet sein mit einem Steuermodul pro UV-Strahlungsquelle oder mit einem Steuermodul für die mindestens eine UV-Strahlungsquelle, insbesondere die Vielzahl an UV-Strahlungsquellen. Besonders bevorzugt handelt es sich hierbei um ein Steuermodul für die UV-Strahlungsquelle bzw. die UV-Strahlungsquellen des Behälterverschlussdeckels des Vorratsbehälters. Ferner hat sich als sehr zweckdienlich erwiesen, auf ein Steuermodul zurückzugreifen, mit dem sämtliche UV-Strahlungsquellen der erfindungsgemäßen Abtönanlage angesteuert werden können.

Als geeignete UV-Strahlungsquellen haben sich UV-LED-Strahlungsquellen hervorgetan. Diese weisen neben dem niedrigeren Energieverbrauch, was Kosten einspart, auch eine geringere Wärmeentwicklung auf. Da gerade Wärme den Bakterienbefall sowie die Bildung von Schimmel begünstigt, ist es vorteilhaft, die Wärmeentwicklung so gering wie möglich zu halten. Ferner ist von Vorteil, dass durch einen verringerten Wärmeeintrag die Antrocknung der Farbton- oder Extenderzusammensetzung und gegebenenfalls auch der Abtönbasiszusammensetzung und damit regelmäßig einhergehende Qualitätseinbußen, wie Stippenbildung, im Endprodukt vermindert bzw. verhindert werden können. Weiterhin sind auch organische Verbindungen, die gegebenenfalls in den Farbton- oder Extenderzusammensetzung enthalten sind, möglicherweise wärme- oder hitzeinstabil.

Grundsätzlich sind als UV-Strahlungsquellen solche besonders geeignet, die UV-Strahlung im Wellenlängenbereich von 100 nm bis 315 nm, bevorzugt im Wellenlängenbereich von 250 nm bis 290 nm, abstrahlen. Hierbei wird besonders bevorzugt auf UV-C-Strahlungsquellen zurückgegriffen, insbesondere solche, die in einem Wellenlängenbereich von 100 bis 280 nm abstrahlen. Insbesondere UV-C-LED-Strahlungsquellen haben eine sehr hohe Energieeffizienz, wobei diese durch Senkung des Fremdlichtanteils noch weniger Abwärme produzieren.

In einer besonders bevorzugten Ausgestaltungsform sind die UV-Strahlungsquellen unabhängig voneinander aktivierbar und/oder, insbesondere und, deaktivierbar. Für den Fall, dass eine Farbton- oder Extenderzusammensetzung in der Abtönanlage zur Neige geht und der Vorratsbehälter nachgefüllt werden muss, können die eine oder die mehreren UV-Strahlungsquellen in diesem Bereich selektiv während der Zwischenreinigung und/oder während des Nachfüllens angefahren werden. Es müssen nicht sämtliche UV-Strahlungsquellen der Abtönanlage ausgeschaltet und wieder eingeschaltet werden, was Zeit und Kosten spart und ebenfalls die Gefahr des Bakterienbefalls und/oder der Schimmelbildung in diesem Nachfüllvorgang minimiert.

Weiterhin haben sich erfindungsgemäße Abtönanlagen als besonders geeignet gezeigt, bei denen die eine oder die mehreren UV-Strahlungsquellen auswechselbare UV-Strahlung-durchlässige Abdeckungen aufweisen. Diese sorgen für eine höhere Lebensdauer der UV-Strahlungsquelle selbst, da die Abdeckungen vor Verschmutzung oder Stöße durch kleinste Partikel schützen. Die auswechselbaren, für UV-Strahlung durchlässigen Abdeckungen sind dabei vorzugsweise aus Quarzglas, Borosilikatglas oder Kunststoff ausgeführt. Unter den geeigneten Kunststoffmaterialien für die für UV-Strahlung durchlässige Abdeckung wird bevorzugt auf Polystyrol, Polymethylmethacrylat, Polycarbonat oder Tetrafluorethylen-Hexafluorpropylen-Copolymere zurückgegriffen. Im Falle von Kunststoff können die Abdeckungen auch als biegsame Kunststofffolie, beispielsweise aus fluorierten Kunststoffen, wie Tetrafluorethylen-Hexafluorpropylen-Copolymeren, ausgebildet sein. Die vorangehend genannten für UV-Strahlung durchlässigen Abdeckungen können in einer Ausführungsform auch, insbesondere integraler, Bestandteil der Behälterverschlussdeckel der Vorratsbehälter für die Farbton- oder Extenderzusammensetzung sein, und zwar insbesondere auch dann, wenn in diesen Behälterverschlussdeckeln die mindestens eine UV-Strahlungsquelle vorliegt. Alternativ oder zusätzlich kann in einer geeigneten Ausführungsformen vorgesehen sein, dass die vorangehend genannten für UV-Strahlung durchlässigen Abdeckungen auch eingerichtet und ausgelegt sind, selber den Vorratsbehälter zu verschließen oder in einer Weise in die Einfüllöffnung des Vorratsbehälters in einer Weise eingepasst werden zu können, dass ein Verrutschen unterbunden wird.

Besonders geeignete erfindungsgemäße Abtönanlagen umfassen oder enthalten ferner eine Farbton- oder Extenderzusammensetzung, insbesondere eine pastöse Pigmentpräparation, in dem mindestens einen Vorratsbehälter für die mindestens eine Farbton- oder Extenderzusammensetzung. Besonders bevorzugt sind Abtönanlagen, die Farbton- oder Extenderzusammensetzungen, insbesondere pastöse Pigmentpräparationen, jeweils in der Mehrzahl, insbesondere in sämtlichen der Vielzahl an Vorratsbehältern für die mindestens eine Farbton- oder Extenderzusammensetzung enthalten. Ganz besonders bevorzugt sind dabei solche erfindungsgemäßen Abtönanlagen, bei denen die Vielzahl an Vorratsbehältern für die mindestens eine Farbton- oder Extenderzusammensetzung auf einem, insbesondere rotierbaren, Karussel angeordnet vorliegt.

Alternativ oder zusätzlich ist es ebenfalls besonders bevorzugt, wenn die Abtönanlage weiterhin eine Abtönbasiszusammensetzung in dem mindestens einen Vorratsbehälter für die Abtönbasiszusammensetzung enthält, oder auch, insbesondere bei Point-of-sale- oder Point-of-use-Anlagen, vor allem solche, die ohne den mindestens einen Vorratsbehälter für die Abtönbasiszusammensetzung auskommen, wenn die Abtönanlage eine Abtönbasiszusammensetzung in dem mindestens einen Mischbehälter für die Abtönbasiszusammensetzung enthält.

Mit der erfindungsgemäßen Abtönanlage gelingt es besonders unproblematisch, die eine oder mehreren Farbton- oder Extenderzusammensetzungen und gegebenenfalls auch die Abtönbasiszusammensetzung, die in der erfindungsgemäßen Abtönanlage gemischt und abgefüllt werden, im Wesentlichen konservierungsmittelfrei einzusetzen. Auf diese Weise können die eingangs thematisierten Umweltrichtlinien besonders zuverlässig eingehalten sowie gesundheitliche Aspekte bei der Handhabung der Abtönanlage berücksichtigt werden. Insbesondere sind die eine oder mehreren Farbton- oder Extenderzusammensetzungen und insbesondere auch die Abtönbasiszusammensetzung frei von organischen bioziden Konservierungsmitteln. Von besonderem Vorteil der erfindungsgemäßen Abtönanlage ist auch, dass damit aus Abtönbasiszusammensetzung und Farbton- bzw. Extenderzusammensetzung erhaltene Beschichtungsstoffe konservierungsmittelfrei zur Verfügung gestellt werden können. Diese Beschichtungsstoffe werden im Sinne der vorliegenden Erfindung im Einklang mit der "Vergabegrundlage für Umweltzeichen" der RAL gGmbH betreffend "Emissionsarme Innenwandfarben RAL-UZ 102" (Ausgabe Januar 2015) regelmäßig als konservierungsmittelfrei angesehen, wenn darin keine Konservierungsmittel oder Konservierungsmittel, auf die Einzelsubstanz bezogen (einschließlich Formaldehyd), in einer Menge kleiner 2 ppm, sowie in Bezug auf CIT (5-Chlor-2-methyl-4-isothiazolin) in einer Menge kleiner 0,5 ppm enthalten sind.

Die der Erfindung zugrunde liegende Aufgabe wird ebenfalls gelöst durch einen Vorratsbehälter für eine Farbton- oder Extenderzusammensetzung, insbesondere enthaltend eine Farbton- oder Extenderzusammensetzung, für eine Abtönanlage, insbesondere die erfindungsgemäße Abtönanlage, umfassend mindestens einen Behältereinlass, eine Behälterwandung umfassend eine Behälterinnenwand, einen Behälterboden und einen Behälterauslass sowie mindestens eine, insbesondere 2, 3, 4 oder mehr UV-Strahlungsquellen, die dem Vorratsbehälter zuordenbar ist bzw. sind, wobei die mindestens eine UV-Strahlungsquelle ausgelegt und eingerichtet ist, um die Behälterinnenwand der Behälterwandung mindestens abschnittsweise sowie gegebenenfalls auch den Behältereinlass, den Behälterboden, in dem Behälter vorliegendes Zubehör, insbesondere ein Rührwerkzeug, eine in dem Vorratsbehälter vorliegende Farbton- oder Extenderzusammensetzung und/oder den Behälterauslass, jeweils mindestens abschnittsweise, insbesondere im Wesentlichen vollflächig, mit UV-Strahlung zu bestrahlen. Dieser erfindungsgemäße Vorratsbehälter ist ausgestattet mit einem Behälterverschlussdeckel, der eingerichtet und ausgelegt ist, um den Vorratsbehälter reversibel zu verschließen, umfassend einen Deckelrand mit einer Innen- und einer Außenseite und vorzugsweise eine sich an den Deckelrand anschließende Deckelseitenwand mit einer Innen- und einer Außenseite, und eine mit dem Deckelrand oder der Deckelseitenwand verbundene Abdeckfläche mit einer Innen- und einer Außenseite, wobei die Innenseite des Deckelrandes und/oder die Innenseite der Deckelseitenwand und/oder die Innenseite der Abdeckfläche, insbesondere die Innenseite der Abdeckfläche, mindestens eine UV-Strahlungsquelle, insbesondere 2, 3 oder 4 UV-Strahlungsquellen, umfasst, die ausgelegt und eingerichtet ist bzw. sind, um die Behälterinnenwand der Behälterwandung des Vorratsbehälters mindestens abschnittsweise sowie gegebenenfalls auch den Behältereinlass, die Innenseite der Deckelseitenwand, den Behälterboden, in dem Behälter vorliegendes Zubehör, insbesondere ein Rührwerkzeug, eine in dem Vorratsbehälter vorliegende Farbton- oder Extenderzusammensetzung und/oder den Behälterauslass mindestens abschnittsweise mit UV-Strahlung zu bestrahlen.

Die der Erfindung zugrunde liegende Aufgabe wird gemäß einer ersten Ausführungsvariante weiterhin gelöst durch ein Verfahren zur Herstellung eines abgetönten Anstrich- oder Putzsystems unter Einsatz der erfindungsgemäßen Abtönanlage, umfassend die Schritte
i) Zurverfügungstellung von mindestens einer Farbton- oder Extenderzusammensetzung in dem mindestens einen Vorratsbehälter für die Farbton- oder Extenderzusammensetzung, insbesondere Zurverfügungstellung einer Vielzahl an Farbton- oder Extenderzusammensetzungen, jeweils in einem der Vielzahl an Vorratsbehältern für die Farbton- oder Extenderzusammensetzungen, wobei diese vorzugsweise auf einem, insbesondere rotierbaren, Karussell angeordnet sind,
ii) Zurverfügungstellung der Abtönbasiszusammensetzung in dem Vorratsbehälter für die Abtönbasiszusammensetzung,
iii) Zusammenführen mindestens einer der Farbton- oder Extenderzusammensetzungen aus dem mindestens einen Vorratsbehälter für die Farbton- oder Extenderzusammensetzung mit der Abtönbasiszusammensetzung, insbesondere über die den Vorratsbehältern jeweils nachgeschalteten Dosierpumpen und Fließkanäle, in dem Mischbehälter,
iv) Vermengen der zusammengeführten Farbtonzusammensetzung oder der Farbtonzusammensetzungen und der Abtönbasiszusammensetzung in dem Mischbehälter unter Erhalt eines abgetönten Anstrich- oder Putzsystems, insbesondere unter Verwendung einer Rüttelplatte oder eines gryroskopischen Mischers, und
v) gegebenenfalls Entnahme des abgetönten Anstrich- oder Putzsystems über den Behälterauslass des Mischbehälters,

wobei die UV-Strahlungsquelle oder die UV-Strahlungsquellen, die dem mindestens einen Vorratsbehälter für eine Farbton- oder Extenderzusammensetzung, insbesondere über dessen Behälterverschlussdeckel, zuordenbar ist bzw. sind, oder
die UV-Strahlungsquelle oder die UV-Strahlungsquellen, die jeweils einem Vorratsbehälter der Vielzahl an Vorratsbehältern für eine Farbton- oder Extenderzusammensetzung, insbesondere über den jeweils korrespondierenden Behälterverschlussdeckel, zuordenbar sind, vor, während und/oder nach dem Befüllen, insbesondere vor, während und nach dem Befüllen, des bzw. der Vorratsbehälter mit der Farbton- oder Extenderzusammensetzung UV-Strahlung emittieren.

Die der Erfindung zugrunde liegende Aufgabe wird gemäß einer weiteren Ausführungsvariante gelöst durch ein Verfahren zur Herstellung eines abgetönten Anstrich- oder Putzsystems unter Einsatz der erfindungsgemäßen Abtönanlage, umfassend die Schritte
a) Zurverfügungstellung von mindestens einer Farbton- oder Extenderzusammensetzung in dem mindestens einen Vorratsbehälter für die Farbton- oder Extenderzusammensetzung, insbesondere Zurverfügungstellung einer Vielzahl an Farbton- oder Extenderzusammensetzungen, jeweils in einem der Vielzahl an Vorratsbehältern für die Farbton- oder Extenderzusammensetzungen, wobei diese vorzugsweise auf einem, insbesondere rotierbaren, Karussell angeordnet sind,
b) Zurverfügungstellung der Abtönbasiszusammensetzung in einem Mischbehälter für die Abtönbasiszusammensetzung,
c) Einfüllen mindestens einer der Farbton- oder Extenderzusammensetzungen, insbesondere über die den jeweiligen Vorratsbehältern nachgeschalteten Dosierpumpen und/oder Fließkanälen, in den Mischbehälter und
d) Vermengen der Farbtonzusammensetzung gemäß a) und der Abtönbasiszusammensetzung gemäß b) in dem Mischbehälter unter Erhalt eines abgetönten Anstrich- oder Putzsystems, insbesondere unter Verwendung einer Rüttelplatte oder eines gyroskopischen Mischers,

wobei die UV-Strahlungsquelle oder die UV-Strahlungsquellen, die dem mindestens einen Vorratsbehälter für eine Farbton- oder Extenderzusammensetzung, insbesondere über dessen Behälterverschlussdeckel, zuordenbar ist bzw. sind, oder
die UV-Strahlungsquelle oder die UV-Strahlungsquellen, die jeweils einem Vorratsbehälter der Vielzahl an Vorratsbehältern für eine Farbton- oder Extenderzusammensetzung, insbesondere über den jeweils korrespondierenden Behälterverschlussdeckel, zuordenbar sind, vor, während und/oder nach dem Befüllen, insbesondere vor, während und nach dem Befüllen, des bzw. der Vorratsbehälter mit der Farbton- oder Extenderzusammensetzung UV-Strahlung emittieren.

Als besonders geeignet hat sich ein Verfahren gezeigt, bei dem die eine oder mehrere UV-Strahlungsquellen, die dem mindestens einen Vorratsbehälter für eine Abtönbasiszusammensetzung zuordenbar ist oder sind, vor, während und/oder nach dem Befüllen des Vorratsbehälters mit der Abtönbasiszusammensetzung UV-Strahlung emittieren.

Ebenfalls ist eine Ausführungsvariante des erfindungsgemäßen Verfahrens besonders vorteilhaft, bei dem die mindestens eine UV-Strahlungsquelle, die dem Fließkanal zuordenbar ist, während und/oder nach dem Durchleiten der Abtönbasiszusammensetzung UV-Strahlung emittiert und/oder bei dem die mindestens eine UV-Strahlungsquelle, die mindestens einem der weiteren, insbesondere jeweils einem der weiteren Fließkanäle, zuordenbar ist, während und/oder nach dem Durchleiten der mindestens einen Farbton- oder Extenderzusammensetzung UV-Strahlung emittiert und/oder bei dem die mindestens eine UV-Strahlungsquelle, die dem Mischbehälter zuordenbar ist, vor, während und/oder nach dem Mischen des abgetönten Anstrich- oder Putzsystems UV-Strahlung emittiert.

In einer zweckmäßigen Ausgestaltungsform des erfindungsgemäßen Verfahrens emittieren die UV-Strahlungsquellen kontinuierlich UV-Strahlung. In einer bevorzugten Ausgestaltungsform des erfindungsgemäßen Verfahrens emittieren die UV-Strahlungsquellen intermittierend UV-Strahlung. Im Falle eines intermittierenden Verlaufs ist dieser vorzugsweise auf den Betriebszyklus der Abtönanlage abgestimmt, sodass beim Hochfahren und der Inbetriebnahme der Anlage besonders auf hohe Keimfreiheit geachtet werden kann.

Als besonders wirksam in Bezug auf die Lösung der der Erfindung zugrunde liegenden Aufgabe hat sich erwiesen, wenn die UV-Strahlungsquelle oder die UV-Strahlungsquellen intervallweise UV-Strahlung emittieren. Als besonders bevorzugt hat sich hierbei eine Pulsrate im Bereich von 0,1 Hz bis 100 MHz, besonders bevorzugt im Bereich von 0,1 MHz bis 10 MHz, herausgestellt. Alternativ ist es selbstverständlich ebenfalls möglich, die UV-Strahlungsquelle bzw. die UV-Strahlungsquellen im Dauerbetrieb strahlen zu lassen.

Die der Erfindung zugrunde liegende Aufgabe wird des Weiteren gelöst durch ein Verfahren zum Lagern mindestens einer Farbton- oder Extenderzusammensetzung in einer erfindungsgemäßen Abtönanlage, wobei man die in dem mindestens einen Vorratsbehälter für die Farbton- oder Extenderzusammensetzung, insbesondere die in der Vielzahl an Vorratsbehälter für Farbton- oder Extenderzusammensetzungen vorliegende bzw. vorliegenden Farbton- oder Extenderzusammensetzungen intervallweise UV-Strahlung aussetzt, bevorzugt mit einer Pulsrate im Bereich von 0,1 Hz bis 100 MHz, besonders bevorzugt mit einer Pulsrate im Bereich von 0,1 MHz bis 10 MHz.

Die der Erfindung zugrunde liegende Aufgabe wird ferner gelöst durch die Verwendung der Abtönanlage für ein Kit-of-Parts-Abtönsystem für die Herstellung, insbesondere konservierungsmittelfreier, abgetönter Anstrich- oder Putzsysteme, insbesondere von abgetönten wässrigen Lasuren, Wasserlacken, lösemittelhaltigen Lasuren, Lösemittellacken, Anstrichfarben, insbesondere Dispersionsfarben, besonders bevorzugt Dispersionsinnenfarben, Kalkfarben, Silikatfarben, Silikonharzfarben, Dispersionssilikatfarben, Sol-Silikat-Farben und/oder Nano-Hybridfarben, insbesondere auf Basis einer wässrigen Organosilikat-Hybrid-Dispersion, Bodenbeschichtungen oder Putzen, insbesondere Silikonharzputze, Silikatputze, Sol-Silikat-Putze, Leichtputze, Organosilikat-Hybriddispersionsputze und/oder Kunstharzputze. Besonders geeignet in der Verwendung sind hierbei Abtönanlagen, die als eine *Point-of-Sale-* oder eine Point-of-Use-Abtönanlage ausgebildet sind.

Mit der erfindungsgemäßen Abtönanlage für ein Kit-of-Parts-Abtönsystem, dem erfindungsgemäßen Verfahren zur Herstellung eines abgetönten Anstrich- oder Putzsystems mit der erfindungsgemäßen Abtönanlage sowie der erfindungsgemäßen Verwendung der Abtönanlage geht die überraschende Erkenntnis einher, dass individuelle Anstriche oder Putze bereitgestellt werden können, ohne diese kostenintensiv und in größeren Mengen lagern zu müssen. Weiterhin verhindert die erfindungsgemäße Abtönanlage die Gefahr des Bakterienbefalls und letztlich auch der Schimmelbildung, insbesondere auch bei den anfälligeren Farben und Lacken auf Wasserbasis. Ein mit der erfindungsgemäßen Abtönanlage einhergehender pragmatischer Vorteil besteht demgemäß auch darin, dass eine sehr ausgeprägte Lagerstabilität verbunden mit einem hohen Maß an Verarbeitungssicherheit in Bezug auf die Farbton- und Extenderzusammensetzungen gewährleistet werden kann. Die erfindungsgemäße Abtönanlage erlaubt ferner die Bereitstellung von Anstrichen und Putzen über einen längeren Zeitraum unter Verzicht auf Biozide. Außerdem gelingt es mit den erfindungsgemäßen Abtönanlagen überraschend, abgetönte Beschichtungsmassen in stets geleichbleibend guter Qualität zur Verfügung zu stellen.

Weitere Merkmale und Vorteile der Erfindung ergeben sich aus der nachfolgenden Beschreibung, in der ein Ausführungsbeispiel der Erfindung anhand einer schematischen Zeichnung beispielhaft erläutert wird, ohne dadurch die Erfindung zu beschränken. Dabei zeigen
- Figur 1: eine schematische Darstellung einer Vorrichtung enthaltend eine erste Ausführungsvariante einer erfindungsgemäßen Abtönanlage,
- Figur 2: eine schematische Darstellung einer Vorrichtung enthaltend eine zweite Ausführungsvariante einer erfindungsgemäßen Abtönanlage,
- Figur 3: eine schematische Ansicht einer dritten Ausführungsvariante einer erfindungsgemäßen Abtönanlage,
- Figur 4: eine schematische Schnittansicht eines erfindungsgemäßen Vorratsbehälters für eine Farbton- oder Extenderzusammensetzung und
- Figur 5: eine schematische Draufsicht auf die Innenseite eines Behälterverschlussdeckels eines erfindungsgemäßen Vorratsbehälters.

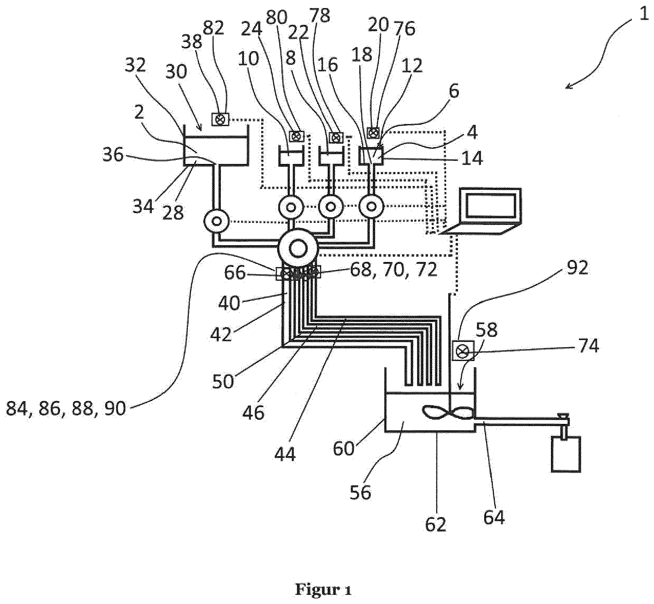

Figur 1 zeigt eine erfindungsgemäße Abtönanlage 1 mit einem Vorratsbehälter 28 für eine Abtönbasiszusammensetzung 2 mit einem Behältereinlass 30, einer Behälterwandung 32, einem Behälterboden 34 und einem Behälterauslass 36 sowie - rein exemplarisch - drei Vorratsbehältern 6, 8 und 10 für hier drei unterschiedliche Farbton- oder Extenderzusammensetzungen 4 mit jeweils - aber exemplarisch nur an Vorratsbehälter 6 gezeigt - einem Behältereinlass 12, einer Behälterwandung 14, einem Behälterboden 16 und einem Behälterauslass 18. Die drei Vorratsbehälter 6, 8 und 10 stehen hier in einer zweckmäßigen Ausführungsform der erfindungsgemäßen Abtönanlage 1 exemplarisch für eine Vielzahl an Vorratsbehältern, beispielsweise im Bereich von 16 bis 32 oder 20 bis 32 Vorratsbehälter. Die Behälterauslässe 36 und 18 der Vorratsbehälter 28, 6, 8 und 10 münden in die Fließkanäle 40, 44, 46 und 50, welche alle eine Innenwandung (hier 42 exemplarisch für den Fließkanal 40 gezeigt) aufweisen. Im oberen Bereich der abgebildeten Vorratsbehälter 28, 6, 8 und 10 bzw. oberhalb der Vorratsbehälter 28, 6, 8 und 10, vorzugsweise in einer Weise, dass jeweils mindestens die Oberflächen der Farbton- oder Extenderzusammensetzungen 4 in diesen Behälter mit UV-Strahlung bestrahlt werden, sind die UV-Strahlungslampen 38, 20, 22 und 24 angebracht, welche in der dargestellten Ausführungsform durch UV-Strahlung-durchlässige Abdeckungen 82, 80, 78 und 76 geschützt sind. Die Fließkanäle 40, 44, 46 und 50, welche in der gezeigten Ausführungsform bevorzugt jeweils eine separate Pumpenvorrichtung aufweisen können, münden in den Mischbehälter 56, der ebenfalls einen Behältereinlass 58, eine Behälterwandung 60, einen Behälterboden 62 und einen Behälterauslass 64 aufweist. In dem Mischbehälter werden die Abtönbasiszusammensetzung 2 und die drei Farbton- oder Extenderzusammensetzungen 4 mittels Rührsystemen vermischt und können dann abgefüllt werden. Im Bereich des Mischbehälters ist ebenfalls eine UV-Strahlungsquelle 74 mit UV-Strahlung-durchlässiger Abdeckung 92 angebracht. Weiterhin können auch im Abfüllkanal weitere UV-Strahlungsquellen angebracht sein. Die UV-Strahlungsquelle, die Pumpen- und die Rührsysteme der Abtönanlage 1 sind über eine Computersoftware steuerbar. Die Ausführungsform der erfindungsgemäßen Abtönanlage 1 gemäß Figur 1 kommt bevorzugt als großindustrielle Fertigungsanlage für die Herstellung großer Mengen an einer abgetönten Farbemasse, welche anschließend auf kleinere Verkaufsgebinde aufgeteilt werden kann, zum Einsatz.

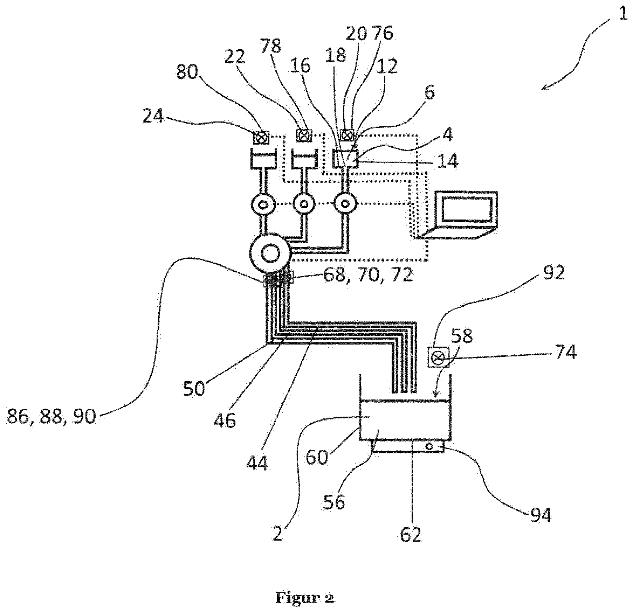

Figur 2 zeigt eine erfindungsgemäße Abtönanlage 1 mit drei Vorratsbehältern 6, 8 und 10 für drei unterschiedliche Farbton- oder Extenderzusammensetzungen 4 mit jeweils - aber exemplarisch nur an Vorratsbehälter 6 gezeigt - einem Behältereinlass 12, einer Behälterwandung 14, einem Behälterboden 16 und einem Behälterauslass 18. Auch die drei Vorratsbehälter 6, 8 und 10 der erfindungsgemäßen Abtönanlage 1 gemäß Figur 2 stehen hier in einer zweckmäßigen Ausführungsform der erfindungsgemäßen Abtönanlage 1 exemplarisch für eine Vielzahl an Vorratsbehältern, beispielsweise im Bereich von 16 bis 32 oder 20 bis 32 Vorratsbehältern. Die Ausführungsform der erfindungsgemäßen Abtönanlage 1 gemäß Figur 2 kommt, anders als die Abtönanlage gemäß Figur 1, ohne den dortigen Vorratsbehälter 28 für eine Abtönbasiszusammensetzung 2 aus. Vielmehr kann in einer Variante die Abtönbasiszusammensetzung 2 in dem Mischbehälter 56, beispielsweise einem Eimer oder einem ähnlichen Gebinde, vorgelegt werden. Die Ausführungsform der erfindungsgemäßen Abtönanlage 1 gemäß Figur 2 kommt bevorzugt als Point-of-sale- oder Point-of-Use-Abtönanlage zum Einsatz. Die Behälterauslässe 18 der Vorratsbehälter 6, 8 und 10 münden in die Fließkanäle 44, 46 und 50, welche alle eine Innenwandung aufweisen. Im oberen Bereich der abgebildeten Vorratsbehälter 6, 8 und 10 bzw. oberhalb der Vorratsbehälter 6, 8 und 10, vorzugsweise in einer Weise, dass jeweils mindestens die Oberflächen der Farbton- oder Extenderzusammensetzungen 4 in diesen Behälter mit UV-Strahlung bestrahlt werden, sind die UV-Strahlungslampen 20, 22 und 24 angebracht, welche in der dargestellten Ausführungsform durch UV-strahlungsdurchlässige Abdeckungen 80, 78 und 76 geschützt sind.

Die Fließkanäle 44, 46 und 50, welche in der gezeigten Ausführungsform bevorzugt jeweils eine separate Pumpenvorrichtung aufweisen können, münden in den Mischbehälter 56, der in der dargestellten Ausführungsform einen Behältereinlass 58, eine Behälterwandung 60, einen Behälterboden 62 aufweist und bei dem es sich z. B. um ein Gebinde wie einen Eimer handeln kann, das nach Vermengen von Farbton- oder Extenderzusammensetzung(en) und Abtönbasiszusammensetzung vom Nutzer bzw. Käufer zum Ort der Verwendung transportiert werden kann. Demgemäß liegt in einer zweckmäßigen Ausführungsform, wie in Figur 2 angedeutet, in dem Mischbehälter 56 die Abtönbasiszusammensetzung 2 vorzugsweise bereits vor und die drei Farbton- oder Extenderzusammensetzungen 4 können über die Fließkanäle 44, 46 und 50 zugegeben werden. Das Vermengen der genannten Komponenten erfolgt in der dargestellten Variante mittels Rütteln bzw. Schütteln. Hierfür kann die Rüttelplatte 94 verwendet werden. Im Bereich des Mischbehälters kann eine UV-Strahlungsquelle 74 mit UV-strahlungsdurchlässiger Abdeckung 92 angebracht sein. Auch bei der Ausführungsform gemäß Figur 2 sind die UV-Strahlungsquelle, die Pumpen- und die Rührsysteme der Abtönanlage 1 bevorzugt über eine Computersoftware steuerbar.

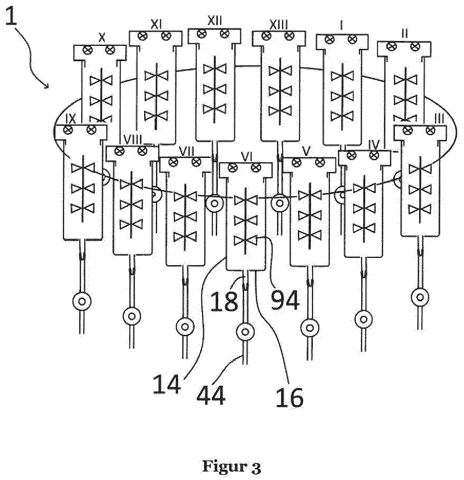

Figur 3 zeigt eine erfindungsgemäße Abtönanlage 1 mit, exemplarisch, dreizehn Vorratsbehältern I bis XIII für dreizehn unterschiedliche Farbton- oder Extenderzusammensetzungen 4 mit jeweils - aber exemplarisch nur an Vorratsbehälter VI gezeigt - einem Behältereinlass 12, einer Behälterwandung 14, einem Behälterboden 16, einem Behälterauslass 18 und einem Rührwerk 92. Die dreizehn Vorratsbehälter I bis XIII stehen hier in einer zweckmäßigen Ausführungsform der erfindungsgemäßen Abtönanlage 1 exemplarisch für eine Vielzahl an Vorratsbehältern, beispielsweise im Bereich von 16 bis 32 oder 20 bis 32 Vorratsbehälter. Die Ausführungsform der erfindungsgemäßen Abtönanlage 1 gemäß Figur 3 zeigt die Anordnung der Vorratsbehältern I bis XIII in einem Karussell, wobei die unterschiedlichen Farbton- oder Extenderzusammensetzungen 4 nacheinander zu einer Abtönbasiszusammensetzung 2 (nicht gezeigt) zugegeben werden. Durch Drehung des Karussells über eine Computer-Software oder eine Bedienungsvorrichtung am Karussell selbst (nicht gezeigt) können nacheinander die verschiedenen Behälterauslasse 18 in Verbindung mit dem jeweiligen Fließkanal 44 angesteuert werden, zweckmäßigerweise indem die jeweilige Pumpenvorrichtung aktiviert wird. Alternativ denkbar sind auch Karussell- oder Plattensysteme, bei denen die zwei, drei oder mehr unterschiedlichen Farbton- oder Extenderzusammensetzungen 4 im Wesentlichen gleichzeitig zugegeben werden, wobei in diesem Fall mehrere Pumpenvorrichtungen gleichzeitig aktiviert werden.

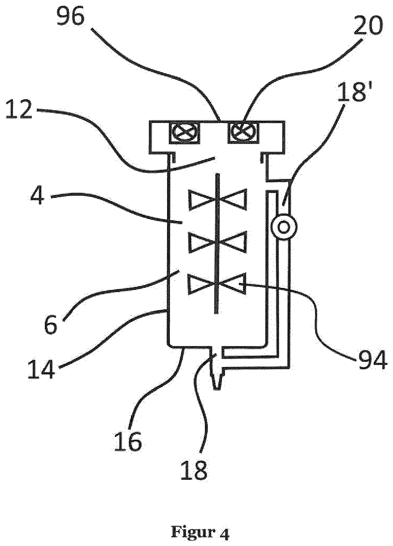

Figur 4 zeigt eine schematische Schnittansicht eines erfindungsgemäßen Vorratsbehälters 6 für eine Farbton- oder Extenderzusammensetzung 4. Dabei weist der Vorratsbehälter 6 einen Behältereinlass 12, eine Behälterwandung 14, einen Behälterboden 16, eine Behälterauslass 18, ein Rührwerk 92 und ein Behälterverschlussdeckel 96 auf. Die gezeigte Ausführungsform zeigt als optionale Komponente zusätzlich eine Abzweigung 18' des Behälterauslasses 18, enthaltend eine Pumpenvorrichtung. Gerade bei Farbton- oder Extenderzusammensetzungen 4, die längere Zeit stehen, können diese so - in Kombination mit dem Rührwerk 92 - ebenfalls über die Aktivierung der Pumpenvorrichtung, welche vorzugsweise im oberen Abschnitt der Abzweigung 18' vorliegt, durchmischt werden, indem die Farbton- oder Extenderzusammensetzungen 4 abgelassen und wieder von oben auf die Oberfläche der Farbton- oder Extenderzusammensetzungen 4 gepumpt wird.

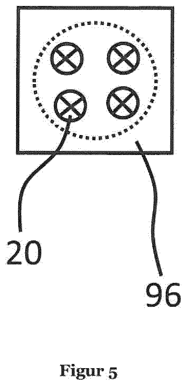

Figur 5 zeigt eine schematische Draufsicht auf die Innenseite eines Behälterverschlussdeckels 96 eines erfindungsgemäßen Vorratsbehälters 6. In der gezeigten Ausführungsform des Behälterverschlussdeckels 96 sind vier UV-Strahlungslampen 20 integriert, die ebenfalls eine UV-Strahlung-durchlässige Abdeckung (nicht gezeigt) aufweisen können,

### Liste der Bezugszeichen

- 1: Abtönanlage
- 2: Abtönbasiszusammensetzung
- 4: Farbton- oder Extenderzusammensetzung
- 6: Vorratsbehälter für Farbton- oder Extenderzusammensetzung
- 8: Vorratsbehälter für Farbton- oder Extenderzusammensetzung
- 10: Vorratsbehälter für Farbton- oder Extenderzusammensetzung
- 12: Behältereinlass des Vorratsbehälters 6 der Farbton- oder Extenderzusammensetzung
- 14: Behälterwandung des Vorratsbehälters 6 der Farbton- oder Extenderzusammensetzung
- 16: Behälterboden des Vorratsbehälters 6 der Farbton- oder Extenderzusammensetzung
- 18: Behälterauslass des Vorratsbehälters 6 der Farbton- oder Extenderzusammensetzung
- 20: UV-Strahlungsquelle des Vorratsbehälters 6
- 22: UV-Strahlungsquelle des Vorratsbehälters 8
- 24: UV-Strahlungsquelle des Vorratsbehälters 10
- 26: UV-Strahlung
- 28: Vorratsbehälter für Abtönbasiszusammensetzung 2
- 30: Behältereinlass des Vorratsbehälters 28 der Abtönbasiszusammensetzung
- 32: Behälterwandung des Vorratsbehälters 28 der Abtönbasiszusammensetzung
- 34: Behälterboden des Vorratsbehälters 28 der Abtönbasiszusammensetzung
- 36: Behälterauslass des Vorratsbehälters 28 der Abtönbasiszusammensetzung
- 38: UV-Strahlungsquelle des Vorratsbehälters 28
- 40: Fließkanal von Vorratsbehälter 28
- 42: Innenwandung des Fließkanals 40
- 44: Fließkanal von Vorratsbehälter 6
- 46: Fließkanal von Vorratsbehälter 8
- 48: Fließkanal von Vorratsbehälter 10
- 50: Innenwandung des Fließkanals 44
- 52: Innenwandung des Fließkanals 46
- 54: Innenwandung des Fließkanals 48
- 56: Mischbehälter
- 58: Behältereinlass des Mischbehälters 56
- 6o: Behälterwandung des Mischbehälters 56
- 62: Behälterboden des Mischbehälters 56
- 64: Behälterauslass des Mischbehälters 56
- 66: UV-Strahlungsquelle des Fließkanals 40
- 68: UV-Strahlungsquelle des Fließkanals 44
- 70: UV-Strahlungsquelle des Fließkanals 46
- 72: UV-Strahlungsquelle des Fließkanals 48
- 74: UV-Strahlungsquelle des Mischbehälters 56
- 76: Abdeckung für UV-Strahlungsquelle 20
- 78: Abdeckung für UV-Strahlungsquelle 22
- 80: Abdeckung für UV-Strahlungsquelle 24
- 82: Abdeckung für UV-Strahlungsquelle 38
- 84: Abdeckung für UV-Strahlungsquelle 66
- 86: Abdeckung für UV-Strahlungsquelle 68
- 88: Abdeckung für UV-Strahlungsquelle 70
- 90: Abdeckung für UV-Strahlungsquelle 72
- 92: Abdeckung für UV-Strahlungsquelle 74
- 94: Rührwerkzeug
- 96: Behälterverschlussdeckel
- I-XIII: Vorratsbehälter für Farbton- oder Extenderzusammensetzung

## Patentansprüche

1. *Point-of-Sale*-Abtönanlage oder *Point-of-Use-*Abtönanlage (1) für ein Kit-of-Parts-Abtönsystem enthaltend eine Abtönbasiszusammensetzung (2) und mindestens eine Farbton- oder Extenderzusammensetzung (4), umfassend
einen Vorratsbehälter (6) für eine Farbton- oder Extenderzusammensetzung (4) oder
eine Vielzahl an Vorratsbehältern (6, 8, 10) für eine Vielzahl an Farbton- oder Extenderzusammensetzungen (4) und
gegebenenfalls einen, zwei oder mehr Vorratsbehälter (28) für eine Abtönbasiszusammensetzung (2) oder für Komponenten einer Abtönbasiszusammensetzung (2),
wobei der oder die Vorratsbehälter (6, 8, 10) mindestens einen Behältereinlass (12), eine Behälterwandung (14); umfassend eine Behälterinnenwand, einen Behälterboden (16) und einen Behälterauslass (18) aufweisen,
ferner enthaltend
einen Behälterverschlussdeckel (96) für einen Vorratsbehälter (6), insbesondere eine Vielzahl an Behälterverschlussdeckeln (96) für die Vielzahl an Vorratsbehältern (6, 8, 10), eingerichtet und ausgelegt, um den einen Vorratsbehälter (6) reversibel zu verschließen, umfassend einen Deckelrand mit einer Innen- und einer Außenseite und vorzugsweise eine sich an den Deckelrand anschließende Deckelseitenwand mit einer Innen- und einer Außenseite, und eine mit dem Deckelrand oder der Deckelseitenwand verbundene Abdeckfläche mit einer Innen- und einer Außenseite,
**gekennzeichnet durch**
mindestens eine, insbesondere 2, 3, 4 oder mehr UV-Strahlungsquellen (20), die dem einen Vorratsbehälter (6) zuordenbar ist bzw. sind oder
mindestens eine, insbesondere 2, 3, 4 oder mehr UV-Strahlungsquellen, die mindestens zwei Vorratsbehältern (6, 8), insbesondere sämtlichen, der Vielzahl an Vorratsbehältern (6, 8, 10) zuordenbar ist bzw. sind oder
mindestens eine, insbesondere 2, 3, 4 oder mehr UV-Strahlungsquellen, die jeweils einem der Vielzahl an Vorratsbehältern (6, 8, 10) zuordenbar ist bzw. sind,
wobei die mindestens eine, insbesondere 2, 3, 4 oder mehr UV-Strahlungsquellen (20, 22, 24) ausgelegt und eingerichtet ist bzw. sind, um die Behälterinnenwand der Behälterwandung (14) mindestens eines Vorratsbehälters (6), insbesondere eines Vorratsbehälters (6), mindestens abschnittsweise sowie gegebenenfalls auch den Behältereinlass (12), den Behälterboden (16), in dem Vorratsbehälter (6) vorliegendes Zubehör, insbesondere ein Rührwerkzeug (94), eine in dem Vorratsbehälter (6) vorliegende Farbton- oder Extenderzusammensetzung (4) und/oder den Behälterauslass (18) mindestens abschnittsweise mit UV-Strahlung (26) zu bestrahlen,
wobei die Innenseite des Deckelrandes und/oder die Innenseite der Deckelseitenwand und/oder die Innenseite der Abdeckfläche die mindestens eine UV-Strahlungsquelle (20), insbesondere 2, 3 oder 4 UV-Strahlungsquellen, umfasst, die ausgelegt und eingerichtet ist bzw. sind, um die Behälterinnenwand der Behälterwandung (14) des Vorratsbehälters (6) mindestens abschnittsweise sowie gegebenenfalls auch den Behältereinlass (12), die Innenseite der Deckelseitenwand, den Behälterboden (16), in dem Vorratsbehälter (6) vorliegendes Zubehör, insbesondere ein Rührwerkzeug (94), eine in dem Vorratsbehälter (6) vorliegende Farbton- oder Extenderzusammensetzung (4) und/oder den Behälterauslass (18) mindestens abschnittsweise mit UV-Strahlung (26) zu bestrahlen.

2. Abtönanlage (1) nach Anspruch 1, ferner enthaltend
ein Steuermodul pro UV-Strahlungsquelle oder
ein Steuermodul für die mindestens eine UV-Strahlungsquelle (20), insbesondere die Vielzahl an UV-Strahlungsquellen, die ausgelegt und eingerichtet ist bzw. sind, um die Behälterinnenwand der Behälterwandung (14) eines Vorratsbehälters (6) mindestens abschnittsweise zu bestrahlen, insbesondere ein Steuermodul für die UV-Strahlungsquelle (20) bzw. die UV-Strahlungsquellen des Behälterverschlussdeckels (96) des Vorratsbehälters (6), oder
ein Steuermodul für sämtliche UV-Strahlungsquellen.

3. Abtönanlage (1) nach einem der vorangehenden Ansprüche, ferner enthaltend mindestens einen mit dem Behälterauslass (18) des mindestens einen Vorratsbehälters (6) der Farbton- oder Extenderzusammensetzungen (4) in Verbindung stehenden Fließkanal (44, 46, 48) und/oder
mindestens einen Mischbehälter (56) mit einem Behältereinlass (58), einer Behälterwandung (60), einem Behälterboden (62) und gegebenenfalls einem Behälterauslass (64) zum Vermengen einer Abtönbasiszusammensetzung (2) mit mindestens einer Farbton- oder Extenderzusammensetzung (4) aus dem mindestens einen Vorratsbehälter (6); und/oder
mindestens ein, insbesondere rotierbares, Karussell, enthaltend die Vielzahl an Vorratsbehältern (I bis XIII) für die Farbton- oder Extenderzusammensetzungen (4) und gegebenenfalls einen, zwei oder mehr Vorratsbehälter (28) für eine Abtönbasiszusammensetzung (2) oder für Komponenten einer Abtönbasiszusammensetzung (2).

4. Abtönanlage (1) nach Anspruch 3, 4-, **gekennzeichnet durch** mindestens eine Dosierpumpe, die über einen Fließkanal (44) mit dem Behälterauslass (18) des mindestens einen Vorratsbehälters (6) einer Farbton- oder Extenderzusammensetzung (4) verbunden ist.

5. Abtönanlage (1) nach einem der vorangehenden Ansprüche, **dadurch gekennzeichnet, dass** die UV-Strahlungsquelle (20, 22, 24, 38, 66, 68, 70, 72, 74) mindestens eine UV-LED-Strahlungsquelle umfasst oder darstellt.

6. Abtönanlage (1) nach einem der vorangehenden Ansprüche, **dadurch gekennzeichnet, dass** die UV-Strahlungsquelle (20, 22, 24, 38, 66, 68, 70, 72, 74) UV-Strahlung im Wellenlängenbereich von 100 nm bis 315 nm, bevorzugt im Wellenlängenbereich von 250 nm bis 290 nm, abstrahlt und insbesondere eine UV-C-Strahlungsquelle darstellt.

7. Abtönanlage (1) nach einem der vorangehenden Ansprüche, **dadurch gekennzeichnet, dass** die UV-Strahlungsquellen (20, 22, 24, 38, 66, 68, 70, 72, 74) unabhängig voneinander aktvierbar und/oder deaktivierbar sind.

8. Abtönanlage (1) nach einem der vorangehenden Ansprüche, **gekennzeichnet durch** eine, insbesondere auswechselbare, für UV-Strahlung-durchlässige Abdeckung (76, 78, 80, 82, 84, 86, 88, 90, 92) für die UV-Strahlungsquelle oder die UV-Strahlungsquellen (20, 22, 24, 38, 66, 68, 70, 72, 74), insbesondere umfassend oder bestehend aus Quarzglas, Borosilikatglas, Polystyrol, Polymethylmethacrylat, Polycarbonat oder Tetrafluorethylen-Hexafluorpropylen-Copolymer.

9. Abtönanlage (1) nach einem der vorangehenden Ansprüche, **dadurch gekennzeichnet, dass** die jeweils mit den Behälterauslässen der Vielzahl an Vorratsbehältern (6, 8, 10) für die Farbton- oder Extenderzusammensetzungen (4) oder die mit den jeweils einem Vorratsbehälter zugeordneten Dosierpumpen in Verbindung stehenden weiteren Fließkanäle (44, 46, 48) eingerichtet und ausgelegt sind, um die Farbton- oder Extenderzusammensetzungen (4) einem Mischbehälter (56) zum Vermengen mit einer darin vorlegbaren oder insbesondere vorliegenden Abtönbasiszusammensetzung (2) zuzuführen, wobei die Abtönanlage (1) vorzugsweise keinen Vorratsbehälter (28) für die Abtönbasiszusammensetzung (2) mit einem Behältereinlass (30), einer Behälterwandung (32), einem Behälterboden (34) und einem Behälterauslass (36) aufweist.

10. Abtönanlage (1) nach einem der vorangehenden Ansprüche, ferner enthaltend eine Farbton- oder Extenderzusammensetzung (4), insbesondere pastöse Pigmentpräparation, in dem mindestens einen Vorratsbehälter (6) für die mindestens eine Farbton- oder Extenderzusammensetzung (4), und insbesondere eine Farbton- oder Extenderzusammensetzung (4), insbesondere pastöse Pigmentpräparation, jeweils in der Mehrzahl, insbesondere in sämtlichen der Vielzahl an Vorratsbehältern (6, 8, 10) für die mindestens eine Farbton- oder Extenderzusammensetzung (4).

11. Abtönanlage (1) nach Anspruch 10, **dadurch gekennzeichnet, dass** die mindestens eine Farbton- oder Extenderzusammensetzung (4) oder die Vielzahl an Farbton- oder Extenderzusammensetzungen (4) im Wesentlichen konservierungsmittelfrei ist bzw. sind.

12. Verfahren zur Herstellung eines abgetönten Anstrich- oder Putzsystems unter Einsatz der Abtönanlage (1) gemäß einem der Ansprüche 1 bis 11, umfassend die Schritte
i) Zurverfügungstellung von mindestens einer Farbton- oder Extenderzusammensetzung (4) in dem mindestens einen Vorratsbehälter (6) für die Farbton- oder Extenderzusammensetzung (4), insbesondere Zurverfügungstellung einer Vielzahl an Farbton- oder Extenderzusammensetzungen (4), jeweils in einem der Vielzahl an Vorratsbehältern (6, 8, 10) für die Farbton- oder Extenderzusammensetzungen (4),
ii) Zurverfügungstellung der Abtönbasiszusammensetzung (2) in dem Vorratsbehälter (28) für die Abtönbasiszusammensetzung (2),
iii) Zusammenführen mindestens einer der Farbton- oder Extenderzusammensetzungen (4) aus dem mindestens einen Vorratsbehälter (6) für die Farbton- oder Extenderzusammensetzung (4) mit der Abtönbasiszusammensetzung (2), insbesondere über die den Vorratsbehältern (6, 28) jeweils nachgeschalteten Dosierpumpen und Fließkanäle (44, 40), in dem Mischbehälter (56),
iv) Vermengen der zusammengeführten Farbton- oder Extenderzusammensetzung (4) oder der Farbton- oder Extenderzusammensetzungen (4) und der Abtönbasiszusammensetzung (2) in dem Mischbehälter (56) unter Erhalt eines abgetönten Anstrich- oder Putzsystems, insbesondere unter Verwendung einer Rüttelplatte oder eines gyroskopischen Mischers, und
v) gegebenenfalls Entnahme des abgetönten Anstrich- oder Putzsystems über den Behälterauslass (64) des Mischbehälters (56),
oder umfassend die Schritte:
a) Zurverfügungstellung von mindestens einer Farbton- oder Extenderzusammensetzung (4) in dem mindestens einen Vorratsbehälter (6) für die Farbton- oder Extenderzusammensetzung (4), insbesondere Zurverfügungstellung einer Vielzahl an Farbton- oder Extenderzusammensetzungen (4), jeweils in einem der Vielzahl an Vorratsbehältern (6, 8, 10) für die Farbton- oder Extenderzusammensetzungen (4),
b) Zurverfügungstellung der Abtönbasiszusammensetzung (2) in einem Mischbehälter (56) für die Abtönbasiszusammensetzung (2),
c) Einfüllen mindestens einer der Farbton- oder Extenderzusammensetzungen (4), insbesondere über die den jeweiligen Vorratsbehältern (6) nachgeschalteten Dosierpumpen und/oder Fließkanälen (44), in den Mischbehälter (56) und
d) Vermengen der Farbton- oder Extenderzusammensetzung (4) gemäß a) und der Abtönbasiszusammensetzung (2) gemäß b) in dem Mischbehälter (56) unter Erhalt eines abgetönten Anstrich- oder Putzsystems, insbesondere unter Verwendung einer Rüttelplatte oder eines gyroskopischen Mischers,
wobei
die UV-Strahlungsquelle (20) oder die UV-Strahlungsquellen, die dem mindestens einen Vorratsbehälter (6) für eine Farbton- oder Extenderzusammensetzung (4), insbesondere über dessen Behälterverschlussdeckel (96), zuordenbar ist bzw. sind, oder
die UV-Strahlungsquelle (20) oder die UV-Strahlungsquellen (20, 22, 24), die jeweils einem Vorratsbehälter der Vielzahl an Vorratsbehältern (6, 8, 10) für eine Farbton- oder Extenderzusammensetzung (4), insbesondere über den jeweils korrespondierenden Behälterverschlussdeckel (96), zuordenbar sind, vor, während und/oder nach dem Befüllen, insbesondere vor, während und nach dem Befüllen, des bzw. der Vorratsbehälter (6) mit der Farbton- oder Extenderzusammensetzung (4) UV-Strahlung (26) emittieren.

13. Verfahren nach Anspruch 12, **dadurch gekennzeichnet, dass** die UV-Strahlungsquelle (38) oder die UV-Strahlungsquellen intervallweise UV-Strahlung (26) emittieren, bevorzugt mit einer Pulsrate im Bereich von 0,1 Hz bis 100 MHz, besonders bevorzugt mit einer Pulsrate im Bereich von 0,1 MHz bis 10 MHz.

14. Verfahren zum Lagern mindestens einer Farbton- oder Extenderzusammensetzung (4) in einer Abtönanlage (1) gemäß einem der Ansprüche 1 bis 11. **dadurch gekennzeichnet, dass** man die in dem mindestens einen Vorratsbehälter (6) für die Farbton- oder Extenderzusammensetzung (4), insbesondere die in der Vielzahl an Vorratsbehälter für Farbton- oder Extenderzusammensetzungen (4) vorliegende bzw. vorliegenden Farbton- oder Extenderzusammensetzungen (4) intervallweise UV-Strahlung (26) aussetzt, bevorzugt mit einer Pulsrate im Bereich von 0,1 Hz bis 100 MHz, besonders bevorzugt mit einer Pulsrate im Bereich von 0,1 MHz bis 10 MHz.

15. Verwendung der Abtönanlage (1) für ein Kit-of-Parts-Abtönsystem nach einem Ansprüche 1 bis 11 für die Herstellung, insbesondere konservierungsmittelfreier, abgetönter Anstrich- oder Putzsysteme, insbesondere von abgetönten wässrigen Lasuren, Wasserlacken, lösemittelhaltigen Lasuren, Lösemittellacken, Anstrichfarben, insbesondere Dispersionsfarben, besonders bevorzugt Dispersionsinnenfarben, Kalkfarben, Silikatfarben, Silikonharzfarben, Dispersionssilikatfarben, Sol-Silikat-Farben und/oder Nano-Hybridfarben, insbesondere auf Basis einer wässrigen Organosilikat-Hybrid-Dispersion, Bodenbeschichtungen oder Putzen, insbesondere Silikonharzputze, Silikatputze, Sol-Silikat-Putze, Leichtputze, Organosilikat-Hybriddispersionsputze und/oder Kunstharzputze.

16. Vorratsbehälter (6) für eine Farbton- oder Extenderzusammensetzung (4), insbesondere enthaltend eine Farbton- oder Extenderzusammensetzung (4), für eine Abtönanlage (1), umfassend mindestens einen Behältereinlass (12), eine Behälterwandung (14) umfassend eine Behälterinnenwand, einen Behälterboden (16) und einen Behälterauslass (18) sowie mindestens eine, insbesondere 2, 3, 4 oder mehr UV-Strahlungsquellen (20), die dem Vorratsbehälter (6) zuordenbar ist bzw. sind, wobei die mindestens eine UV-Strahlungsquelle (20, 22, 24) ausgelegt und eingerichtet ist, um die Behälterinnenwand der Behälterwandung (14) mindestens abschnittsweise sowie gegebenenfalls auch den Behältereinlass (12), den Behälterboden (16), in dem Behälter vorliegendes Zubehör, insbesondere ein Rührwerkzeug (94), eine in dem Vorratsbehälter (6) vorliegende Farbton- oder Extenderzusammensetzung (4) und/oder den Behälterauslass (18), jeweils mindestens abschnittsweise mit UV-Strahlung (26) zu bestrahlen, ferner enthaltend einen Behälterverschlussdeckel (96),eingerichtet und ausgelegt, um den einen Vorratsbehälter (6) reversibel zu verschließen, umfassend einen Deckelrand mit einer Innen- und einer Außenseite und vorzugsweise eine sich an den Deckelrand anschließende Deckelseitenwand mit einer Innen- und einer Außenseite, und eine mit dem Deckelrand oder der Deckelseitenwand verbundene Abdeckfläche mit einer Innen- und einer Außenseite, wobei die Innenseite des Deckelrandes und/oder die Innenseite der Deckelseitenwand und/oder die Innenseite der Abdeckfläche, insbesondere die Innenseite der Abdeckfläche, mindestens eine UV-Strahlungsquelle (20), insbesondere 2, 3 oder 4 UV-Strahlungsquellen, umfasst, die ausgelegt und eingerichtet ist bzw. sind, um die Behälterinnenwand der Behälterwandung (14) des Vorratsbehälters (6) mindestens abschnittsweise sowie gegebenenfalls auch den Behältereinlass (12), die Innenseite der Deckelseitenwand, den Behälterboden (16), in dem Behälter vorliegendes Zubehör, insbesondere ein Rührwerkzeug (94), eine in dem Vorratsbehälter (6) vorliegende Farbton- oder Extenderzusammensetzung (4) und/oder den Behälterauslass (18) mindestens abschnittsweise mit UV-Strahlung (26) zu bestrahlen.

## Claims

1. A point-of-sale tinting system or a point-of-use tinting system (1) for a kit-of-parts tinting system, containing a basic tinting composition (2) and at least one color or extender composition (4), comprising
a supply container (6) for a color or extender composition (4) or
a plurality of supply containers (6, 8, 10) for a plurality of color or extender compositions (4) and,
if applicable, one, two or more supply containers (28) for a basic tinting composition (2) or for components of a basic tinting composition (2),
wherein the supply container(s) (6, 8, 10) has/have at least one container inlet (12), a container wall (14); comprising a container inner wall, a container bottom (16) and a container outlet (18), further containing a container closure lid (96) for a supply container (6), in particular a plurality of container closure lids (96) for the plurality of supply containers (6, 8,10), adapted and arranged to reversibly close the one supply container (6), comprising a lid edge having an inner and an outer side and, preferably, a lid side wall having an inner and an outer side joining the lid edge, and a cover surface having an inner and an outer side connected to the lid edge or the lid side wall,
**characterized by**
at least one UV radiation source, in particular 2, 3, 4 or more UV radiation sources (20), which can be assigned to the one supply container (6) or
at least one UV radiation source, in particular 2, 3, 4 or more UV radiation sources, which can be assigned to the at least two supply containers (6, 8), in particular all, of the plurality of supply containers (6, 8, 10) or
at least one UV radiation source, in particular 2, 3, 4 or more UV radiation sources, which can in each case be assigned to one of the plurality of supply containers (6, 8, 10),
wherein the at least one UV radiation source, in particular 2, 3, 4 or more UV radiation sources (20, 22, 24), is or are adapted and arranged to irradiate the inner wall of the container wall (14) of at least one supply container (6), in particular of a supply container (6), at least in sections, and, if applicable, also the container inlet (12), the container bottom (16), accessories present in the supply container (6), in particular a stirring tool (94), a color or extender composition (4) present in the supply container (6) and/or the container outlet (18), at least in sections, with UV radiation (26),
wherein the inner side of the lid edge and/or the inner side of the lid side wall and/or the inner side of the cover surface comprise(s) the at least one UV radiation source (20), in particular 2, 3 or 4 UV radiation sources, which is or are adapted and arranged to irradiate the inner wall of the container wall (14) of the supply container (6), at least in sections, and, if applicable, also the container inlet (12), the inner side of the lid side wall, the container bottom (16), accessories present in the supply container (6), in particular a stirring tool (94), a color or extender composition (4) present in the supply container (6) and/or the container outlet (18), at least in sections, with UV radiation (26).

2. The tinting system (1) according to Claim 1, further containing a control module for each UV radiation source or a control module for the at least one UV radiation source (20), in particular the plurality of UV radiation sources, which is or are adapted and arranged to irradiate the inner wall of the container wall (14) of a supply container (6), at least in sections, in particular a control module for the UV radiation source (20) or the UV radiation sources of the container closure lid (96) of the supply container (6), or a control module for all UV radiation sources.

3. The tinting system (1) according to any one of the preceding claims, further containing at least one flow channel (44, 46, 48) in connection with the container outlet (18) of the at least one supply container (6) of the color or extender compositions (4) and/or
at least one mixing container (56) having a container inlet (58), a container wall (60), a container bottom (62) and, if applicable, a container outlet (64) for blending a basic tinting composition (2) with at least one color or extender composition (4) from the at least one supply container (6); and/or
at least one, in particular rotatable, carousel, containing the plurality of supply containers (I to XIII) for the color or extender compositions (4) and, if applicable, one, two or more supply containers (28) for a basic tinting composition (2) or for components of a basic tinting composition (2).

4. The tinting system (1) according to Claim 3, **characterized by**
at least one dosing pump which is connected by way of a flow channel (44) to the container outlet (18) of the at least one supply container (6) of a color or extender composition (4).

5. The tinting system (1) according to any one of the preceding claims, **characterized in that**
the UV radiation source (20, 22, 24, 38, 66, 68, 70, 72, 74) comprises or constitutes at least one UV LED radiation source.

6. The tinting system (1) according to any one of the preceding claims, **characterized in that**
the UV radiation source (20, 22, 24, 38, 66, 68, 70, 72, 74) radiates UV radiation in the wavelength range of 100 nm to 315 nm, preferably in the wavelength range of 250 nm to 290 nm, and in particular constitutes a UV-C radiation source.

7. The tinting system (1) according to any one of the preceding claims, **characterized in that**
the UV radiation sources (20, 22, 24, 38, 66, 68, 70, 72, 74) can be activated and/or deactivated independently of one another.

8. The tinting system (1) according to any one of the preceding claims, **characterized by** a cover (76, 78, 80, 82, 84, 86, 88, 90, 92), which is permeable to UV radiation and which can in particular be exchanged, for the UV radiation source or the UV radiation sources (20, 22, 24, 38, 66, 68, 70, 72, 74), in particular comprising or consisting of quartz glass, borosilicate glass, polystyrene, polymethyl methacrylate, polycarbonate or tetrafluoroethylene/hexafluoropropylene copolymer.

9. The tinting system (1) according to any one of the preceding claims, **characterized in that**
the further fluid channels (44, 46, 48) which are, in each case, connected to the container outlets of the plurality of supply containers (6, 8, 10) for the color or extender compositions (4) or the dosing pumps assigned to the, in each case, one supply container are adapted and arranged to feed the color or extender compositions (4) to a mixing container (56) for blending with a basic tinting composition (2) which can be made available therein or which is in particular present therein, wherein the tinting system (1) preferably does not have a supply container (28) having a container inlet (30), a container wall (32), a container bottom (34) or a container outlet (36) for the basic tinting composition (2).

10. The tinting system (1) according to any one of the preceding claims, further containing a color or extender composition (4), in particular a paste-like pigment preparation, in the at least one supply container (6) for the at least one color or extender composition (4), and in particular a color or extender composition (4), in particular a paste-like pigment preparation, in each case in the majority, in particular in all of the plurality of supply containers (6, 8, 10) for the at least one color or extender composition (4).

11. The tinting system (1) according to Claim 10, **characterized in that** the at least one color or extender composition (4) or the plurality of color or extender compositions (4) is or are substantially free of preserving agents.

12. A method for producing a tinted paint or plaster system, utilizing the tinting system (1) according to any one of Claims 1 to 11,
comprising the steps of
i) providing at least one color or extender composition (4) in the at least one supply container (6) for the color or extender composition (4), in particular providing a plurality of color or extender compositions (4), in each case in one of the plurality of supply containers (6, 8, 10) for the color or extender compositions (4),
ii) providing the basic tinting composition (2) in the supply container (28) for the basic tinting composition (2),
iii) combining at least one of the color or extender compositions (4) from the at least one supply container (6) for the color or extender composition (4) with the basic tinting composition (2), in particular by way of the dosing pumps and fluid channels (44, 40) downstream of the supply containers (6, 28) in each case, in the mixing container (56),
iv) blending the combined color or extender composition (4) or the color or extender compositions (4) and the basic tinting composition (2) in the mixing container (56), obtaining a tinted paint or plaster system, in particular using a vibratory plate or a gyroscopic mixer, and
v) if applicable, extracting the tinted paint or plaster system by way of the container outlet (64) of the mixing container (56),
or comprising the steps of:
a) providing at least one color or extender composition (4) in the at least one supply container (6) for the color or extender composition (4), in particular providing a plurality of color or extender compositions (4), in each case in one of the plurality of supply containers (6, 8, 10) for the color or extender compositions (4),
b) providing the basic tinting composition (2) in a mixing container (56) for the basic tinting composition (2),
c) filling at least one of the color or extender compositions (4), in particular by way of the dosing pumps and/or fluid channels (44) downstream of the respective supply containers (6), in the mixing container (56) and
d) blending the color or extender composition (4) according to a) and the basic tinting composition (2) according to b) in the mixing container (56), obtaining a tinted paint or plaster system, in particular using a vibratory plate or a gyroscopic mixer,
wherein
the UV radiation source (20) or the UV radiation sources, which can be assigned to the at least one supply container (6) for a color or extender composition (4), in particular by way of the container closure lid (96) thereof,
or
the UV radiation source (20) or the UV radiation sources (20, 22, 24), which can in each case be assigned to a supply container of the plurality of supply containers (6, 8, 10) for a color or extender composition (4), in particular by way of the corresponding container closure lid (96) in each case, prior to, during and/or following the filling, in particular prior to, during and/or following the filling, of the supply container(s) (6) with the color or extender composition (4), emit UV radiation (26).

13. The method according to Claim 12, **characterized in that**
the UV radiation source (38) or the UV radiation sources emit UV radiation (26) in intervals, preferably with a pulse rate in the range of 0.1 Hz to 100 MHz, particularly preferably with a pulse rate in the range of 0.1 MHz to 10 MHz.

14. A method for storing at least one color or extender composition (4) in a tinting system (1) according to any one of Claims 1 to 11, **characterized in that**
the color or extender composition (4) present in the at least one supply container (6) for the color or extender composition (4), in particular the color or extender compositions (4) present in the plurality of supply containers for color or extender compositions (4), is/are exposed to UV radiation (26) in intervals, preferably with a pulse rate in the range of 0.1 Hz to 100 MHz, particularly preferably with a pulse rate in the range of 0.1 MHz to 10 MHz.

15. Use of the tinting system (1) for a kit-of-parts tinting system according to any one of Claims 1 to 11 for the production of tinted paint or plaster systems, which are in particular free of preserving agents, in particular of tinted aqueous glazes, water varnishes, solvent-containing glazes, solvent-based varnishes, paints, in particular dispersion paints, particularly preferably interior dispersion paints, limewash paints, silicate paints, silicone resin paints, silicate dispersion paints, sol silicate paints and/or nano-hybrid paints, in particular based on an aqueous organosilicate hybrid dispersion, floor coatings or plasters, in particular silicone resin plasters, silicate plasters, sol silicate plasters, lightweight plasters, organosilicate hybrid dispersion plasters and/or synthetic resin plasters.

16. A supply container (6) for a color or extender composition (4), in particular containing a color or extender composition (4), for a tinting system (1), comprising at least one container inlet (12), a container wall (14) comprising a container inner wall, a container bottom (16) and a container outlet (18) and
at least one radiation source, in particular 2, 3, 4 or more UV radiation sources (20), which can be assigned to the supply container (6), wherein the at least one UV radiation source (20, 22, 24) is adapted and arranged to irradiate the inner wall of the container wall (14), at least in sections, and, if applicable, also the container inlet (12), the container bottom (16), accessories present in the container, in particular a stirring tool (94), a color or extender composition (4) present in the supply container (6) and/or the container outlet (18), in each case at least in sections, with UV radiation (26), further containing
a container closure lid (96), adapted and arranged to reversibly close the one supply container (6), comprising a lid edge having an inner and an outer side and, preferably, a lid side wall having an inner and an outer side joining the lid edge, and a cover surface having an inner and an outer side connected to the lid edge or the lid side wall, wherein the inner side of the lid edge and/or the inner side of the lid side wall and/or the inner side of the cover surface, in particular the inner side of the cover surface, comprises at least one UV radiation source (20), in particular 2, 3 or 4 UV radiation sources, which is or are adapted and arranged to irradiate the inner wall of the container wall (14) of the supply container (6), at least in sections, and, if applicable, also the container inlet (12), the inner side of the lid side wall, the container bottom (16), accessories present in the container, in particular a stirring tool (94), a hue or extender composition (4) present in the supply container (6) and/or the container outlet (18), at least in sections, with UV radiation (26).

## Revendications

1. Installation à teinter de *point de vente* ou installation à teinter de *point d'utilisation* (1) pour un système à teinter en kit, contenant une composition de base à teinter (2) et au moins une composition de ton ou de matière de charge (4), comprenant
un récipient de stockage (6) pour une composition de ton ou de matière de charge (4) ou
une pluralité de récipients de stockage (6, 8, 10) pour une pluralité de compositions de tons ou de matières de charge (4) et
le cas échéant, un, deux ou plusieurs récipients de stockage (28) pour une composition de base à teinter (2) ou pour des composants d'une composition de base à teinter (2),
le ou les récipients de stockage (6, 8, 10) présentant au moins une admission de récipient (12), une paroi de récipient (14) ; comprenant une paroi intérieure de récipient, un fond de récipient (16) et une sortie de récipient (18), comprenant en outre un couvercle de fermeture de récipient (96) pour un récipient de stockage (6), en particulier une pluralité de couvercles de fermeture de récipients (96) pour la pluralité de récipients de stockage (6, 8, 10), adaptés et agencés afin de fermer de façon réversible l'un récipient de stockage (6), comprenant un bord de couvercle avec un côté intérieur et un côté extérieur et de préférence une paroi latérale de couvercle étant contiguë au bord de couvercle avec un côté intérieur et un côté extérieur, et une surface de recouvrement reliée au bord de couvercle ou à la paroi latérale de couvercle avec un côté intérieur et un côté extérieur,
**caractérisée par**
au moins une, en particulier 2, 3, 4 ou plus, sources de rayonnement UV (20), laquelle est ou lesquelles sont attribuables à l'un récipient de stockage (6), ou
au moins une, en particulier 2, 3, 4 ou plus, sources de rayonnement UV, laquelle est ou lesquelles sont attribuables à au moins deux récipients de stockage (6, 8), en particulier à l'ensemble de la pluralité de récipients de stockage (6, 8, 10), ou
au moins une, en particulier 2, 3, 4 ou plus, sources de rayonnement UV, laquelle est ou lesquelles sont attribuables respectivement à l'un des récipients parmi la pluralité de récipients de stockage (6, 8, 10),
l'au moins une, en particulier les 2, 3, 4 ou plus sources de rayonnement UV (20, 22, 24) étant adaptées et agencées afin d'irradier avec un rayonnement UV (26) la paroi intérieure de récipient de la paroi de récipient (14) d'au moins un récipient de stockage (6), en particulier d'un récipient de stockage (6), au moins par sections, ainsi que, le cas échéant, également l'admission de récipient (12), le fond de récipient (16), des accessoires présents dans le récipient de stockage (6), en particulier un agitateur (94), une composition de ton ou de matière de charge (4) présente dans le récipient de stockage (6) et/ou la sortie de récipient (18), au moins par sections,
le côté intérieur du bord de couvercle et/ou le côté intérieur de la paroi latérale de couvercle et/ou le côté intérieur de la surface de recouvrement comprenant l'au moins une source de rayonnement UV (20), en particulier 2, 3 ou 4 sources de rayonnement UV, lesquelles sont adaptées et agencées afin d'irradier avec un rayonnement UV (26) la paroi intérieure de récipient de la paroi de récipient (14) du récipient de stockage (6), au moins par sections, ainsi que, le cas échéant, également l'admission de récipient (12), le côté intérieur de la paroi latérale de couvercle, le fond de récipient (16), des accessoires présents dans le récipient de stockage (6), en particulier un agitateur (94), une composition de ton ou de matière de charge (4) présente dans le récipient de stockage (6) et/ou la sortie de récipient (18), au moins par sections.

2. Installation à teinter (1) selon la revendication 1, contenant en outre un module de commande par source de rayonnement UV ou un module de commande pour l'au moins une source de rayonnement UV (20), en particulier la pluralité de sources de rayonnement UV, laquelle est ou lesquelles sont adaptées et agencées afin d'irradier la paroi intérieure de récipient de la paroi de récipient (14) d'un récipient de stockage (6), au moins par sections, en particulier un module de commande pour la source de rayonnement UV (20) ou les sources de rayonnement UV du couvercle de fermeture de récipient (96) du récipient de stockage (6), ou un module de commande pour l'ensemble des sources de rayonnement UV.

3. Installation à teinter (1) selon l'une quelconque des revendications précédentes, contenant en outre au moins un canal d'écoulement (44, 46, 48) relié avec la sortie de récipient (18) de l'au moins un récipient de stockage (6) des compositions de tons ou de matières de charge (4) et/ou
au moins un récipient de mélange (56) avec une admission de récipient (58), une paroi de récipient (60), un fond de récipient (62) et, le cas échéant, une sortie de récipient (64) pour le mélange d'une composition de base à teinter (2) avec au moins une composition de ton ou de matière de charge (4) de l'au moins un récipient de stockage (6) ; et/ou
au moins un carrousel, en particulier rotatif, comprenant la pluralité de récipients de stockage (I à XIII) pour les compositions de tons ou de matières de charge (4) et, le cas échéant, un, deux ou plusieurs récipients de stockage (28) pour une composition de base à teinter (2) ou pour les composants d'une composition de base à teinter (2).

4. Installation à teinter (1) selon la revendication 3, **caractérisée par**
au moins une pompe de dosage, laquelle est reliée par un canal d'écoulement (44) à la sortie de récipient (18) de l'au moins un récipient de stockage (6) d'une composition de ton ou de matière de charge (4).

5. Installation à teinter (1) selon l'une quelconque des revendications précédentes, **caractérisée en ce que**
la source de rayonnement UV (20, 22, 24, 38, 66, 68, 70, 72, 74) comprend ou représente au moins une source de rayonnement UV à LED.

6. Installation à teinter (1) selon l'une quelconque des revendications précédentes, **caractérisée en ce que**
la source de rayonnement UV (20, 22, 24, 38, 66, 68, 70, 72, 74) émet un rayonnement UV dans la gamme de longueurs d'onde de 100 nm à 315 nm, de préférence dans la gamme de longueur d'ondes de 250 nm à 290 nm, et représente en particulier une source de rayonnement UV-c.

7. Installation à teinter (1) selon l'une quelconque des revendications précédentes, **caractérisée en ce que**
les sources de rayonnement UV (20, 22, 24, 38, 66, 68, 70, 72, 74) sont activables et/ou désactivables indépendamment les unes des autres.

8. Installation à teinter (1) selon l'une quelconque des revendications précédentes, **caractérisée par** une couverture perméable au rayonnement UV (76, 78, 80, 82, 84, 86, 88, 90, 92), en particulier interchangeable, pour la source de rayonnement UV ou les sources de rayonnement UV (20, 22, 24, 38, 66, 68, 70, 72, 74), en particulier comprenant ou se composant de verre de quartz, de verre borosilicate, de polystyrène, de polyméthacrylate de méthyle, de polycarbonate ou de copolymère de tétrafluoroéthylène et d'hexafluoropropylène.

9. Installation à teinter (1) selon l'une quelconque des revendications précédentes, **caractérisée en ce que**
les canaux d'écoulement (44, 46, 48) supplémentaires respectivement reliés aux sorties de récipient de la pluralité de récipients de stockage (6, 8, 10) pour les compositions de tons ou de matières de charge (4) ou les canaux d'écoulement (44, 46, 48) supplémentaires reliés aux pompes de dosage attribuées respectivement à l'un récipient de stockage sont adaptés et agencés afin d'amener les compositions de tons ou de matières de charge (4) à un récipient de mélange (56) pour le mélange avec une composition de base à teinter (2) pouvant y être mise ou en particulier s'y trouvant, l'installation à teinter (1) ne présentant de préférence aucun récipient de stockage (28) pour la composition de base à teinter (2) avec une admission de récipient (30), une paroi de récipient (32), un fond de récipient (34) et une sortie de récipient (36).

10. Installation à teinter (1) selon l'une quelconque des revendications précédentes, contenant en outre une composition de ton ou de matière de charge (4), en particulier une préparation de pigments pâteuse, dans l'au moins un récipient de stockage (6) pour l'au moins une composition de ton ou de matière de charge (4), et en particulier une composition de ton ou de matière de charge (4), en particulier une préparation de pigments pâteuse, respectivement une multiplicité, en particulier dans l'ensemble de la pluralité des récipients de stockage (6, 8, 10) pour l'au moins une composition de ton ou de matière de charge (4).

11. Installation à teinter (1) selon la revendication 10, **caractérisée en ce que** l'au moins une composition de ton ou de matière de charge (4) ou la pluralité de compositions de tons ou de matières de charge (4) sont essentiellement exemptes d'agents de conservation.

12. Procédé pour la fabrication d'un système de peinture ou de nettoyage teinté avec utilisation de l'installation à teinter (1) selon l'une des revendications 1 à 11
comprenant les étapes de :
i) mise à disposition d'au moins une composition de ton ou de matière de charge (4) dans l'au moins un récipient de stockage (6) pour la composition de ton ou de matière de charge (4), en particulier mise à disposition d'une pluralité de compositions de tons ou de matières de charge (4), respectivement dans l'un des récipients parmi la pluralité de récipients de stockage (6, 8, 10) pour les compositions de tons ou de matières de charge (4),
ii) mise à disposition de la composition de base à teinter (2) dans le récipient de stockage (28) pour la composition de base à teinter (2),
iii) réunion d'au moins une composition parmi les compositions de tons ou de matières de charge (4) de l'au moins un récipient de stockage (6) pour la composition de ton ou de matière charge (4) avec la composition de base à teinter (2), en particulier par les pompes de dosage et les canaux d'écoulement (44, 40) respectivement en aval des récipients de stockage (6, 28), dans le récipient de mélange (56),
iv) mélange de la composition de ton ou de matière de charge (4) ou des compositions de tons ou de matières de charge (4) et de la composition de base à teinter (2) dans le récipient de mélange (56) avec obtention d'un système de peinture ou de nettoyage teinté, en particulier avec utilisation d'une plaque vibrante ou d'un mélangeur gyroscopique, et
v) le cas échéant, prélèvement du système de peinture ou de nettoyage teinté par la sortie de récipient (64) du récipient de mélange (56),
ou comprenant les étapes de :
a) mise à disposition d'au moins une composition de ton ou de matière de charge (4) dans l'au moins un récipient de stockage (6) pour la composition de ton ou de matière de charge (4), en particulier mise à disposition d'une pluralité de compositions de tons ou de matières de charge (4), respectivement dans l'un des récipients parmi la pluralité de récipients de stockage (6, 8, 10) pour les compositions de tons ou de matières de charge (4),
b) mise à disposition de la composition de base à teinter (2) dans un récipient de stockage (56) pour la composition de base à teinter (2),
c) remplissage d'au moins une composition parmi les compositions de tons ou de matières de charge (4), en particulier par les pompes de dosage et/ou les canaux d'écoulement (44) en aval des récipients de stockage respectifs (6), dans le récipient de mélange (56), et
d) mélange de la composition de ton ou de matière de charge (4) conformément à a) et de la composition de base à teinter (2) conformément à b) dans le récipient de mélange (56), avec obtention d'un système de peinture ou de nettoyage teinté, en particulier avec utilisation d'une plaque vibrante ou d'un mélangeur gyroscopique,
la source de rayonnement UV (20) ou les sources de rayonnement UV, laquelle est ou lesquelles sont attribuables à l'au moins un récipient de stockage (6) pour une composition de ton ou de matière de charge (4), en particulier par son ou leur couvercle de fermeture de récipient (96),
ou
la source de rayonnement UV (20) ou les sources de rayonnement UV (20, 22, 24), laquelle est ou lesquelles sont attribuables à respectivement un récipient de stockage parmi la pluralité de récipients de stockage (6, 8, 10) pour une composition de ton ou de matière de charge (4), en particulier par le couvercle de fermeture de récipient (96) correspondant respectivement, avant, pendant et/ou après le remplissage, en particulier avant, pendant et après le remplissage du ou des récipients de stockage (6) avec la composition de ton ou de matière de charge (4), émettant un rayonnement UV (26).

13. Procédé selon la revendication 12, **caractérisé en ce que**
la source de rayonnement UV (38) ou les sources de rayonnement UV émettent un rayonnement UV (26) par intervalles, de préférence avec un taux d'impulsion dans la plage de 0,1 Hz à 100 MHz, en particulier de préférence avec un taux d'impulsion dans la plage de 0,1 MHz à 10 MHz.

14. Procédé pour le stockage d'au moins une composition de ton ou de matière de charge (4) dans une installation à teinter (1) selon l'une des revendications 1 à 11, **caractérisé en ce que**
l'on soumet par intervalle à un rayonnement UV (26) la composition ou les compositions de tons ou de matière de charge (4) dans l'au moins un récipient de stockage (6) pour la composition de ton ou de matière charge (4), en particulier les compositions de tons ou de matières de charge (4) présentes dans la pluralité de récipients de stockage pour les compositions de tons ou de matières de charge (4), de préférence avec un taux d'impulsion dans la plage de 0,1 Hz à 100 MHz,
en particulier de préférence avec un taux d'impulsion dans la plage de 0,1 MHz à 10 MHz.

15. Utilisation de l'installation à teinter (1) pour un système à teinter en kit selon l'une des revendications 1 à 11 pour la fabrication de systèmes de peinture ou de nettoyage teintés, en particulier exempts d'agents de conservation, en particulier de glacis aqueux, vernis aqueux, glacis à base de solvants, vernis à base de solvants, peintures, en particulier peintures à dispersion, particulièrement de préférence peintures d'intérieur à dispersion, peintures à la chaux, peintures aux silicates, peintures à la résine de silicone, peintures aux silicates à dispersion, peintures sol-silicate et/ou peintures nano-hybrides, en particulier à base d'une dispersion hybride à organosilicates aqueuse, revêtements de sols ou enduits, en particulier enduits à la résine de silicone, enduits silicates, enduits sol-silicate, enduits légers, enduits de dispersion hybrides à organosilicates et/ou enduits à résine synthétique, teintés.

16. Récipient de stockage (6) pour une composition de ton ou de matière de charge (4), en particulier contenant une composition de ton ou de matière de charge (4), pour une installation à teinter (1), comprenant
au moins une admission de récipient (12), une paroi de récipient (14) comprenant une paroi intérieure de récipient, un fond de récipient (16) et une sortie de récipient (18) ainsi qu' au moins une, en particulier 2, 3, 4 ou plus, sources de rayonnement UV (20), laquelle est ou lesquelles sont attribuables au récipient de stockage (6), l'au moins une source de rayonnement UV (20, 22, 24) étant adaptée et agencée afin d'irradier avec un rayonnement UV (26) respectivement la paroi intérieure de récipient de la paroi de récipient (14), au moins par sections, ainsi que, le cas échéant, également l'admission de récipient (12), le fond de récipient (16), des accessoires présents dans le récipient, en particulier un agitateur (94), une composition de ton ou de matériau de charge (4) présente dans le récipient de réserve (6) et/ou la sortie de récipient (18), au moins par sections, comprenant en outre un couvercle de fermeture de récipient (96), adapté et agencé afin de fermer de façon réversible l'un récipient de stockage (6), comprenant un bord de couvercle avec un côté intérieur et un côté extérieur et de préférence une paroi latérale de couvercle étant contiguë au bord de couvercle avec un côté intérieur et un côté extérieur, et une surface de recouvrement reliée au bord de couvercle ou à la paroi latérale de couvercle avec un côté intérieur et un côté extérieur, le côté intérieur du bord de couvercle et/ou le côté intérieur de la paroi latérale de couvercle et/ou le côté intérieur de la surface de recouvrement, en particulier le côté intérieur de la source de recouvrement comprenant au moins une source de rayonnement UV (20), en particulier 2, 3 ou 4 sources de rayonnement UV, laquelle est ou lesquelles sont adaptées et agencées afin d'irradier avec un rayonnement UV (26) la paroi intérieure de récipient de la paroi de récipient (14) du récipient de stockage (6) au moins partiellement ainsi que, le cas échéant, également l'admission de récipient (12), le côté intérieur de la paroi latérale de couvercle, le fond de récipient (16), des accessoires présents dans le récipient, en particulier un agitateur (94), une composition de ton ou de matière de charge (4) présente dans le récipient de stockage (6) et/ou la sortie de récipient (18), au moins par sections.
